# EUROPEAN PATENT APPLICATION

(11) **EP 4 635 952 A1**
(43) Date of publication of application: **22.10.2025**
(21) Application number: 23902558.8
(22) Date of filing: 06.12.2023
(51) Int. Cl.: C07D 409/12, C07D 307/52, A61P 29/00, A61P 1/00, A61P 1/04, A61K 31/341, A61K 31/343, A61K 31/428

(54) **SOLID FORM OF N-SUBSTITUTED PHENYLSULFONAMIDE COMPOUND**

(30) Priority: 15.12.2022 CN 202211617185
(71) Applicant: Shanghai Leado Pharmatech Co. Ltd., Shanghai 201114 (CN)
(72) Inventor: WANG, Youxin, Shanghai 201114 (CN); ZHANG, Lingling, Shanghai 201114 (CN); DING, Qiang, Shanghai 201114 (CN)
(74) Representative: Mewburn Ellis LLP
(86) International application number: PCT/CN2023/136809
(87) International publication number: WO 2024/125361

(57) **Abstract**

A solid form of an N-substituted phenylsulfonamide compound. The present invention specifically relates to a free crystal form, fumarate crystal form, hydrochloride crystal form, sulfate crystal form, succinate crystal form, malate crystal form, phosphate crystal form, tartrate crystal form, pyroglutamate crystal form, benzene sulfonate crystal form, malonate crystal form and hemifumaric acid crystal form of an N-substituted phenylsulfonamide compound. The solid form has excellent stability and solubility, an excellent inhibitory effect on transient receptor potential channel protein TRPA1, and a good therapeutic effect on TRPA1-associated diseases.

## Description

### Technical Field

The present invention relates to the field of pharmaceuticals, and specifically relates to a solid form of *N*-substituted phenylsulfonamide compound.

### Background Technology

TRPA1 is a member of the TRP ion channel superfamily and the only member of the TRPA subfamily. It is a non-selective cation channel and is permeable to Na⁺, K⁺, Ca²⁺ and Mg²⁺. TRPA1 is mainly distributed on the primary sensory neurons of the dorsal root nerve (DRG), trigeminal nerve (TG) and vagus nerve (VG). From the distribution of the human system, TRPA1 is highly expressed in the peripheral nervous system, respiratory system, gastrointestinal system and urinary system. When these organs and tissues have abnormal functions, the expression and function of TRPA1 is usually abnormal simultaneously. TRPA1 can convert cold stimulation, chemical stimulation and mechanical stimulation into inward currents, trigger a series of physiological functions, and participate in the formation of various pain sensations. Inflammatory pain is common problem of certain chronic diseases, and there is still no effective treatment method in the clinic. Animal experimental studies have shown that TRPA1 is involved in inflammatory response and plays an important role in inflammatory pain. The use of TRPA1 specific blockers can significantly reduce the inflammatory pain response in rats. From the current research, TRPA1 plays an important role in the occurrence of asthma and cough. Compounds that induce asthma and cough, either cellular endogenous factors or exogenous factors, can activate TRPA1. TRPA1 antagonists can reduce asthma symptoms and block airway hyperresponsiveness. It is confirmed that TRPA1 participates in the regulation of visceral hypersensitivity and plays an important role in visceral pain through different animal models for visceral hypersensitivity, such as colitis, rectal dilation or stress. Neuropathic pain is a pain syndrome caused by central or peripheral nervous system damage or disease, mainly manifested as hyperalgesia, allodynia, and spontaneous pain. In recent years, more and more studies have shown that TRPA1 channels play an important role in different neurogenic pain, such as diabetic neuropathy and neuropathy caused by chemotherapy drugs. Recent studies have also shown that TRPA1 also has a mediating role in toothache, migraine and other pains. The administration of TRPA1 antagonists can significantly alleviate the pain symptoms.

TRPA1 is widely distributed and expressed in the human system. In addition to the aforementioned physiological functions of TRPA1, the development of TRPA1 inhibitor indications reported so far also involves inflammatory bowel disease, chronic obstructive pulmonary disease, antitussive, antipruritic, allergic rhinitis, ear disease, diabetic, urinary incontinence, etc. TRPA1 is a proven new target for the treatment of many diseases.

N-substituted phenylsulfonamide compound by inhibiting the transient receptor potential ankyrin 1 (TRPA1) exhibits potent efficacy in treating inflammatory bowel disease and pain, showing an excellent safety profile.

### Summary of The Invention

The present invention provides a solid form of N-substituted phenylsulfonamide compound with superior stability and solubility.

In the first aspect, the present invention provides a solid form of *N*-(2-(furan-2-yl)-4-((methylamino)methyl)phenyl)thiophene-3-sulfonamide.

In another preferred embodiment, the compound *N*-(2-(furan-2-yl)-4-((methylamino)methyl)phenyl)thiophene-3-sulfonamide has a structure of formula I:

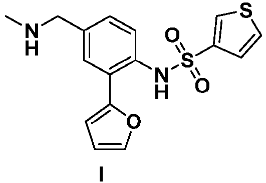

In another preferred embodiment, the solid form of *N*-(2-(furan-2-yl)-4-((methylamino)methyl)phenyl)thiophene-3-sulfonamide includes free crystal form or salt crystal form.

In another preferred embodiment, the salt crystal form of *N*-(2-(furan-2-yl)-4-((methylamino)methyl)phenyl)thiophene-3-sulfonamide is formed by reacting the compound with a pharmaceutically acceptable acid, and the pharmaceutically acceptable acid is selected from the group consisting of hydrochloric acid, sulfuric acid, maleic acid, phosphoric acid, fumaric acid, *L*-tartaric acid, citric acid, *D*-glucuronic acid, *L*-malic acid, succinic acid, pyroglutamic acid, *p*-toluenesulfonic acid, methanesulfonic acid, benzenesulfonic acid, acetic acid, malonic acid, benzoic acid, and hippuric acid.

In another preferred embodiment, the fumarate crystal form is a mono-fumarate crystal form.

In another preferred embodiment, the fumarate crystal form is a hemifumarate crystal form.

In another preferred embodiment, the hydrochloride crystal form is a mono-hydrochloride crystal form.

In another preferred embodiment, the sulfate crystal form is a mono-sulfate crystal form.

In another preferred embodiment, the succinate crystal form is a mono-succinate crystal form.

In another preferred embodiment, the malate crystal form is a mono-malate crystal form.

In another preferred embodiment, the phosphate crystal form is a mono-phosphate crystal form.

In another preferred embodiment, the tartrate crystal form is a mono-tartrate crystal form.

In another preferred embodiment, the pyroglutamate crystal form is a mono-pyroglutamate crystal form.

In another preferred embodiment, the benzene sulfonate crystal form is a mono-benzene sulfonate crystal form.

In another preferred embodiment, the malonate crystal form is a mono-malonate crystal form.

In another preferred embodiment, the solid form is free crystal form A, and the X-ray powder diffraction (XRPD) pattern of the free crystal form A has characteristic peaks at the following 2θ values: 16.24±0.2°, 19.23±0.2°, 23.17±0.2°, 24.45±0.2°, and 32.76±0.2°.

In another preferred embodiment, the free crystal form A has characteristic peaks at the following 2θ values: 12.27±0.2°, 13.55±0.2°, 16.24±0.2°, 18.71±0.2°, 19.23±0.2°, 21.37±0.2°, 22.78±0.2°, 23.17±0.2°, 24.45±0.2°, 25.60±0.2°, and 32.76±0.2°.

In another preferred embodiment, the X-ray powder diffraction pattern of the free crystal form A has the following characteristic peaks and peak intensities with 2θ values as shown below:

| 2θ | d value | Relative intensity (%) |
|---|---|---|
| 10.3508 | 8.55 | 3.91 |
| 12.2743 | 7.21 | 5.71 |
| 12.7358 | 6.95 | 2.74 |
| 13.0498 | 6.78 | 3.37 |
| 13.5519 | 6.53 | 5.11 |
| 14.5598 | 6.08 | 3.29 |
| 16.2435 | 5.46 | 12.37 |
| 16.9947 | 5.22 | 2.47 |
| 18.7105 | 4.74 | 5.18 |
| 19.2294 | 4.62 | 19.76 |
| 20.5157 | 4.33 | 3.72 |
| 21.3650 | 4.16 | 4.74 |
| 22.7773 | 3.90 | 8.18 |
| 23.1731 | 3.84 | 14.42 |
| 24.4467 | 3.64 | 100.00 |
| 25.6010 | 3.48 | 4.09 |
| 25.9685 | 3.43 | 3.63 |
| 26.5486 | 3.36 | 3.12 |
| 27.2523 | 3.27 | 1.16 |
| 28.4973 | 3.13 | 0.82 |
| 29.1448 | 3.06 | 0.55 |
| 31.2276 | 2.86 | 1.09 |
| 32.0955 | 2.79 | 1.51 |
| 32.7583 | 2.73 | 12.62 |
| 33.7741 | 2.65 | 1.44 |
| 36.6494 | 2.45 | 1.14 |
| 38.6393 | 2.33 | 0.90 |
| 38.9834 | 2.31 | 0.96. |

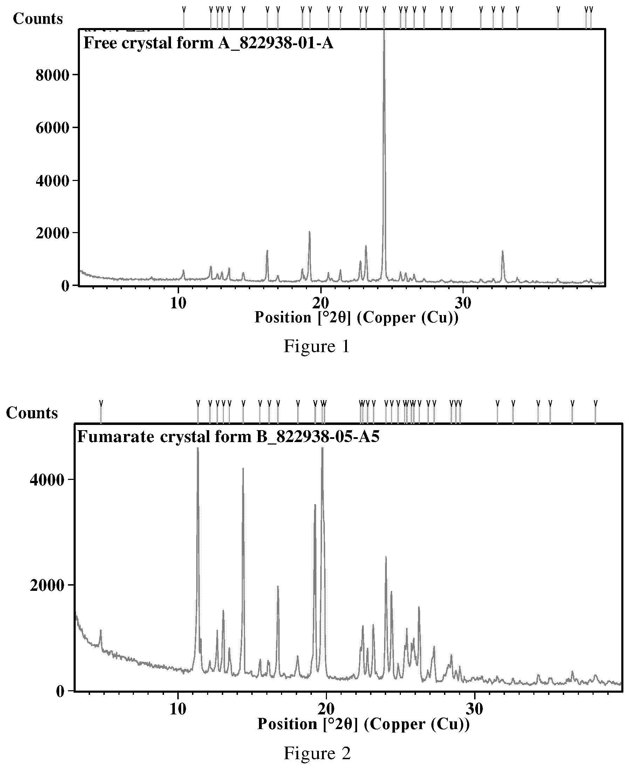

In another preferred embodiment, the free crystal form A has an XRPD pattern substantially as shown in Figure 1.

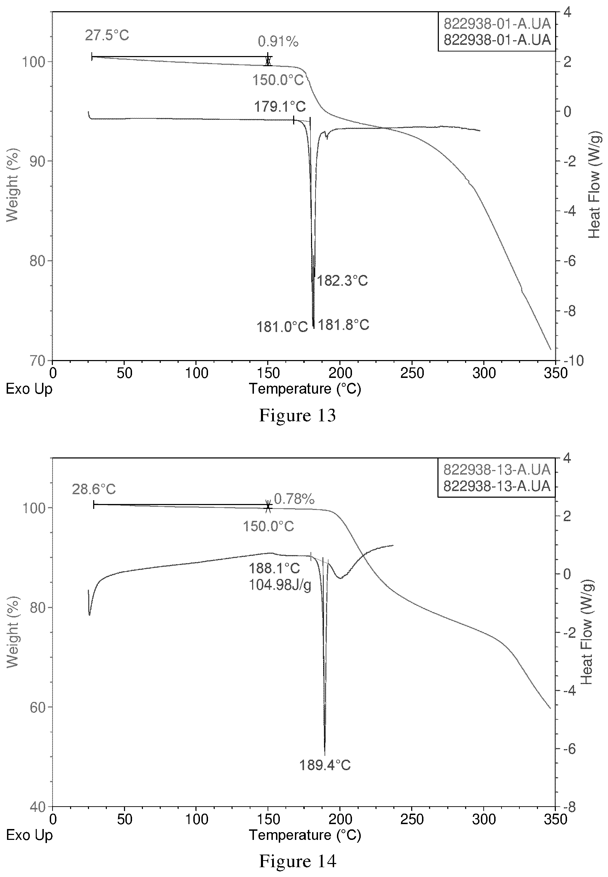

In another preferred embodiment, the free crystal form A includes one or more of the following characteristics:
The differential scanning calorimetry (DSC) curve of the free crystal form A having endothermic peaks at 181.0±5°C (preferably ±4°C, ±3°C, ±2°C, or ±1°C), 181.8±5°C (preferably ±4°C, ±3°C, ±2°C, or ±1°C), and 182.3±5°C (preferably ±4°C, ±3°C, ±2°C, or ±1°C);
The DSC curve of the free crystal form A substantially as shown in Figure 13;
The thermogravimetric analysis (TGA) curve of the free crystal form A showing a weight loss of about 0.9±0.5% (preferably ±0.4%, ±0.3%, ±0.2%, or ±0.1%) at 150°C;
The TGA curve of the free crystal form A substantially as shown in Figure 13.

In another preferred embodiment, the salt crystal forms include fumarate crystal form, hydrochloride crystal form, sulfate crystal form, succinate crystal form, malate crystal form, phosphate crystal form, tartrate crystal form, pyroglutamate crystal form, benzene sulfonate crystal form, malonate crystal form, and hemifumarate crystal form.

In another preferred embodiment, the solid form is fumarate crystal form B, and the XRPD pattern of the fumarate crystal form B has characteristic peaks at the following 2θ values: 11.34±0.2°, 14.40±0.2°, 19.71±0.2°, and 19.86±0.2°.

In another preferred embodiment, the fumarate crystal form B has characteristic peaks at the following 2θ values: 11.34±0.2°, 14.40±0.2°, 19.23±0.2°, 19.71±0.2°.

In another preferred embodiment, the fumarate crystal form B has characteristic peaks at the following 2θ values: 11.34±0.2°, 14.40±0.2°, 16.75±0.2°, 19.23±0.2°, 19.71±0.2°, 19.86±0.2°, 24.03±0.2°.

In another preferred embodiment, the fumarate crystal form B has characteristic peaks at the following 2θ values: 4.79±0.2°, 11.34±0.2°, 12.14±0.2°, 12.64±0.2°, 13.05±0.2°, 13.45±0.2°, 14.40±0.2°, 15.54±0.2°, 16.14±0.2°, 16.75±0.2°, 18.08±0.2°, 19.23±0.2°, 19.71±0.2°, 19.86±0.2°, 22.30±0.2°, 22.47±0.2°, 22.78±0.2°, 23.18±0.2°, 24.03±0.2°, 24.41±0.2°, 24.85±0.2°, 25.28±0.2°, 25.44±0.2°, 25.75±0.2°, 25.91±0.2°, 26.28±0.2°, 26.86±0.2°, 27.28±0.2°, 28.45±0.2°, 28.76±0.2°, 29.02±0.2°, 31.54±0.2°, 32.60±0.2°, 34.30±0.2°, 35.11±0.2°, 36.61±0.2°, 38.17±0.2°.

In another preferred embodiment, the X-ray powder diffraction pattern of the fumarate crystal form B has the following characteristic peaks and peak intensities with 2θ values as shown below:

| 2θ | d value | Relative intensity (%) |
|---|---|---|
| 4.7853 | 18.47 | 7.44 |
| 11.3360 | 7.81 | 95.62 |
| 12.1412 | 7.29 | 3.80 |
| 12.6401 | 7.00 | 17.06 |
| 13.0507 | 6.78 | 24.77 |
| 13.4538 | 6.58 | 10.32 |
| 14.3983 | 6.15 | 80.83 |
| 15.5359 | 5.70 | 6.50 |
| 16.1411 | 5.49 | 5.45 |
| 16.7535 | 5.29 | 34.94 |
| 18.0815 | 4.91 | 8.61 |
| 19.2348 | 4.61 | 67.67 |
| 19.7074 | 4.50 | 100.00 |
| 19.8575 | 4.47 | 51.16 |
| 22.2983 | 3.99 | 12.31 |
| 22.4667 | 3.96 | 20.37 |
| 22.7769 | 3.90 | 12.17 |
| 23.1803 | 3.84 | 20.68 |
| 24.0261 | 3.70 | 47.03 |
| 24.4094 | 3.65 | 34.43 |
| 24.8487 | 3.58 | 6.41 |
| 25.2842 | 3.52 | 12.97 |
| 25.4446 | 3.50 | 20.09 |
| 25.7526 | 3.46 | 14.15 |
| 25.9064 | 3.44 | 16.01 |
| 26.2756 | 3.39 | 28.27 |
| 26.8582 | 3.32 | 4.14 |
| 27.2796 | 3.27 | 13.01 |
| 28.4514 | 3.14 | 10.46 |
| 28.7628 | 3.10 | 4.24 |
| 29.0176 | 3.08 | 6.10 |
| 31.5419 | 2.84 | 2.31 |
| 32.5956 | 2.75 | 1.81 |
| 34.2989 | 2.61 | 3.71 |
| 35.1125 | 2.56 | 2.28 |
| 36.6111 | 2.45 | 4.94 |
| 38.1686 | 2.36 | 2.94. |

In another preferred embodiment, the fumarate crystal form B has an XRPD pattern substantially as shown in Figure 2.

In another preferred embodiment, the fumarate crystal form crystal B includes one or more of the following characteristics:
The DSC curve of the fumarate crystal form B having an endothermic peak at 189.4±5°C (preferably ±4°C, ±3°C, ±2°C, or ±1°C);
The DSC curve of the fumarate crystal form B substantially as shown in Figure 14;
The TGA curve of the fumarate crystal form B showing a weight loss of about 0.8±0.2% (preferably ±0.15%, ±0.1%, ±0.05%, or ±0.02%) at 150°C;
The TGA curve of the fumarate crystal form B substantially as shown in Figure 14.

In another preferred embodiment, the fumarate crystal form B is composed of *N*-(2-(furan-2-yl)-4-((methylamino)methyl)phenyl)thiophene-3-sulfonamide and fumaric acid in a molar ratio of 1-1.5:1-1.5, preferably 1-1.2:1-1.2, and more preferably 1:1.

In another preferred embodiment, the solid form is hydrochloride crystal form C, and the XRPD pattern of the hydrochloride crystal form C has characteristic peaks at the following 2θ values: 17.20±0.2°, 20.34±0.2°, 24.74±0.2°, and 25.25±0.2°.

In another preferred embodiment, the hydrochloride crystal form C has characteristic peaks at the following 2θ values: 11.56±0.2°, 17.20±0.2°, 20.34±0.2°, 23.27±0.2°, 23.52±0.2°, 24.74±0.2°, and 25.25±0.2°.

In another preferred embodiment, the hydrochloride crystal form C has the following characteristic peaks at the following 2θ value: 11.56±0.2°: 13.49±0.2°, 17.20±0.2°, 19.63±0.2°, 20.34±0.2°, 23.27±0.2°, 23.52±0.2°, 24.74±0.2°, 25.25±0.2°.

In another preferred embodiment, the X-ray powder diffraction pattern of the hydrochloride crystal form C has the following characteristic peaks and peak intensities with 2θ values as shown below:

| 2θ | d value | Relative intensity (%) |
|---|---|---|
| 5.7200 | 15.45 | 17.27 |
| 11.5634 | 7.65 | 41.03 |
| 12.3017 | 7.20 | 17.99 |
| 12.5546 | 7.05 | 10.68 |
| 13.4901 | 6.56 | 24.98 |
| 15.5870 | 5.69 | 11.15 |
| 17.2012 | 5.16 | 57.03 |
| 18.5812 | 4.78 | 6.28 |
| 19.6324 | 4.52 | 21.15 |
| 19.9419 | 4.45 | 19.54 |
| 20.3449 | 4.37 | 46.68 |
| 21.7316 | 4.09 | 3.33 |
| 22.2954 | 3.99 | 3.07 |
| 22.6986 | 3.92 | 16.28 |
| 23.2678 | 3.82 | 28.29 |
| 23.5211 | 3.78 | 38.88 |
| 24.1472 | 3.69 | 9.29 |
| 24.5064 | 3.63 | 12.86 |
| 24.7389 | 3.60 | 41.17 |
| 25.2526 | 3.53 | 100.00 |
| 27.1600 | 3.28 | 13.12 |
| 27.5883 | 3.23 | 8.87 |
| 28.3363 | 3.15 | 10.35 |
| 29.6184 | 3.02 | 18.15 |
| 30.5745 | 2.92 | 2.69 |
| 31.5019 | 2.84 | 5.16 |
| 32.2586 | 2.78 | 4.79 |
| 34.2101 | 2.62 | 2.77 |
| 34.8483 | 2.57 | 2.61 |
| 35.5568 | 2.52 | 5.45 |
| 37.2395 | 2.41 | 2.93 |
| 37.8099 | 2.38 | 2.10 |
| 38.4821 | 2.34 | 1.72 |
| 39.0207 | 2.31 | 0.63. |

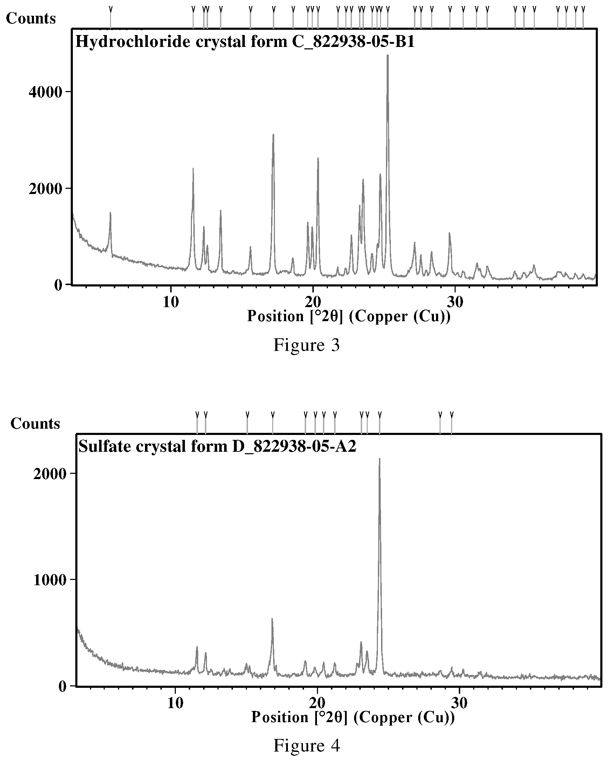

In another preferred embodiment, the hydrochloride crystal form C has an XRPD pattern substantially as shown in Figure 3.

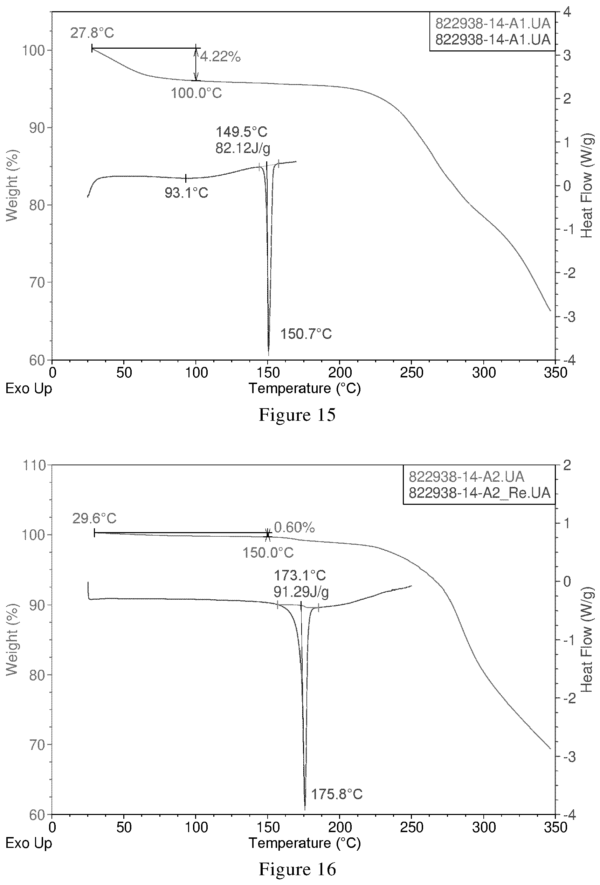

In another preferred embodiment, the hydrochloride crystal form C includes one or more of the following characteristics:
The DSC curve of the hydrochloride crystal form C having endothermic peaks at 93.1±5°C (preferably ±4°C, ±3°C, ±2°C, or ±1°C) and 150.7±5°C (preferably ±4°C, ±3°C, ±2°C, or ±1°C);
The DSC curve of the hydrochloride crystal form C substantially as shown in Figure 15;
The TGA curve of the hydrochloride crystal form C showing a weight loss of about 4.2±0.5% (preferably ±0.4%, ±0.3%, ±0.2%, or ±0.1%) at 100°C;
The TGA curve of the hydrochloride crystal form C substantially as shown in Figure 15.

In another preferred embodiment, the hydrochloride crystal form C is composed of *N*-(2-(furan-2-yl)-4-((methylamino)methyl)phenyl)thiophene-3-sulfonamide and hydrochloric acid in a molar ratio of 1-1.5:1-1.5, preferably 1-1.2:1-1.2, and more preferably 1:1.

In another preferred embodiment, the solid form is sulfate crystal form D, wherein the XRPD pattern of the sulfate crystal form D has characteristic peaks at the following 2θ values: 16.84±0.2°, 23.08±0.2°, and 24.38±0.2°.

In another preferred embodiment, the sulfate crystal form D has characteristic peaks at the following 2θ values: 11.51±0.2°, 12.13±0.2°, 16.84±0.2°, 23.08±0.2°, 23.49±0.2°, and 24.38±0.2°.

In another preferred embodiment, the sulfate crystal form D has characteristic peaks at the following 2θ values: 11.51±0.2°, 12.13±0.2°, 16.84±0.2°, 19.14±0.2°, 20.42±0.2°, 23.08±0.2°, 23.49±0.2°, and 24.38±0.2°.

In another preferred embodiment, the X-ray powder diffraction pattern of the sulfate crystal form D has the following characteristic peaks and peak intensities with 2θ values as shown below:

| 2θ | d value | Relative intensity (%) |
|---|---|---|
| 11.5141 | 7.69 | 11.47 |
| 12.1280 | 7.30 | 9.26 |
| 15.0641 | 5.88 | 2.81 |
| 16.8395 | 5.27 | 22.93 |
| 19.1425 | 4.64 | 6.24 |
| 19.8417 | 4.47 | 3.29 |
| 20.4232 | 4.35 | 5.69 |
| 21.2298 | 4.19 | 4.92 |
| 23.0785 | 3.85 | 13.89 |
| 23.4929 | 3.79 | 10.59 |
| 24.3760 | 3.65 | 100.00 |
| 28.6343 | 3.12 | 2.34 |
| 29.4467 | 3.03 | 3.32. |

In another preferred embodiment, the sulfate crystal form D has an XRPD pattern substantially as shown in Figure 4.

In another preferred embodiment, the sulfate crystal form D includes one or more of the following characteristics:
The DSC curve of the sulfate crystal form D having an endothermic peak at 175.8±5°C (preferably ±4°C, ±3°C, ±2°C, or ±1°C);
The DSC curve of the sulfate crystal form D substantially as shown in Figure 16;
The TGA curve of the sulfate crystal form D showing a weight loss of about 0.6±0.1% (preferably ±0.08%, ±0.05%, ±0.02%, or ±0.01%) at 150°C;
The TGA curve of the sulfate crystal form D substantially as shown in Figure 16.

In another preferred embodiment, the sulfate crystal form D is composed of *N*-(2-(furan-2-yl)-4-((methylamino)methyl)phenyl)thiophene-3-sulfonamide and sulfuric acid in a molar ratio of 1-1.5:1-1.5, preferably 1-1.2:1-1.2, and more preferably 1:1.

In another preferred embodiment, the solid form is succinate crystal form E, wherein the XRPD pattern of the succinate crystal form E has characteristic peaks at the following 2θ values: 12.57±0.2°, 19.18±0.2°, 19.89±0.2°, and 22.68±0.2°.

In another preferred embodiment, the succinate crystal form E has the following characteristic peaks at the following 2θ value: 12.57±0.2°, 14.89±0.2°, 19.18±0.2°, 19.89±0.2°, 22.68±0.2°, 23.48±0.2°, and 24.31±0.2°.

In another preferred embodiment, the succinate crystal form E has the following characteristic peaks at the following 2θ value: 12.57±0.2°, 12.83±0.2°, 14.63±0.2°, 14.89±0.2°, 15.43±0.2°, 18.04±0.2°, 18.35±0.2°, 18.97±0.2°, 19.18±0.2°, 19.89±0.2°, 21.98±0.2°, 22.68±0.2°, 22.97±0.2°, 23.48±0.2°, 24.31±0.2°, 25.15±0.2°, 25.51±0.2°, 26.57±0.2°, and 27.33±0.2°.

In another preferred embodiment, the X-ray powder diffraction pattern of the succinate crystal form E has the following characteristic peaks and peak intensities with 2θ values as shown below:

| 2θ | d value | Relative intensity % |
|---|---|---|
| 11.3012 | 7.83 | 4.51 |
| 12.1022 | 7.31 | 29.55 |
| 12.5682 | 7.04 | 100.00 |
| 12.8251 | 6.90 | 15.88 |
| 14.6341 | 6.05 | 12.00 |
| 14.8890 | 5.95 | 41.52 |
| 15.4251 | 5.74 | 28.69 |
| 16.8216 | 5.27 | 3.36 |
| 17.6695 | 5.02 | 5.00 |
| 18.0351 | 4.92 | 14.38 |
| 18.3497 | 4.84 | 15.48 |
| 18.9745 | 4.68 | 31.08 |
| 19.1778 | 4.63 | 76.32 |
| 19.8916 | 4.46 | 52.13 |
| 21.5010 | 4.13 | 3.16 |
| 21.9794 | 4.04 | 22.25 |
| 22.3705 | 3.97 | 7.90 |
| 22.6780 | 3.92 | 66.97 |
| 22.9693 | 3.87 | 20.66 |
| 23.4757 | 3.79 | 50.03 |
| 24.3137 | 3.66 | 42.93 |
| 25.1547 | 3.54 | 17.11 |
| 25.5114 | 3.49 | 13.68 |
| 25.8422 | 3.45 | 6.55 |
| 26.5689 | 3.36 | 20.74 |
| 27.3275 | 3.26 | 13.09 |
| 27.8768 | 3.20 | 6.11 |
| 28.5630 | 3.13 | 1.87 |
| 29.5834 | 3.02 | 3.10 |
| 30.0966 | 2.97 | 11.09 |
| 31.1706 | 2.87 | 1.74 |
| 31.9845 | 2.80 | 6.84 |
| 33.2344 | 2.70 | 3.96 |
| 34.3892 | 2.61 | 8.84 |
| 34.8567 | 2.57 | 3.40 |
| 36.8940 | 2.44 | 6.24 |
| 38.3053 | 2.35 | 1.89 |
| 38.9442 | 2.31 | 0.78. |

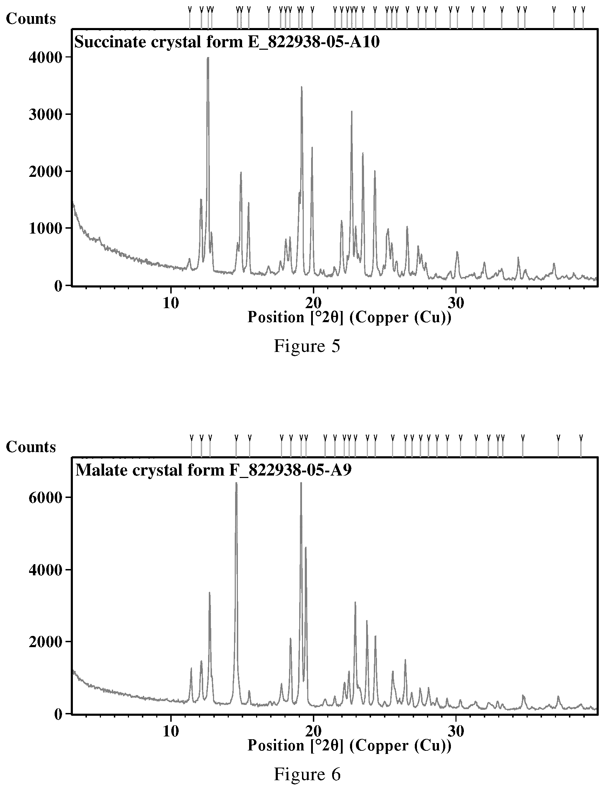

In another preferred embodiment, the succinate crystal form E has an XRPD pattern substantially as shown in Figure 5.

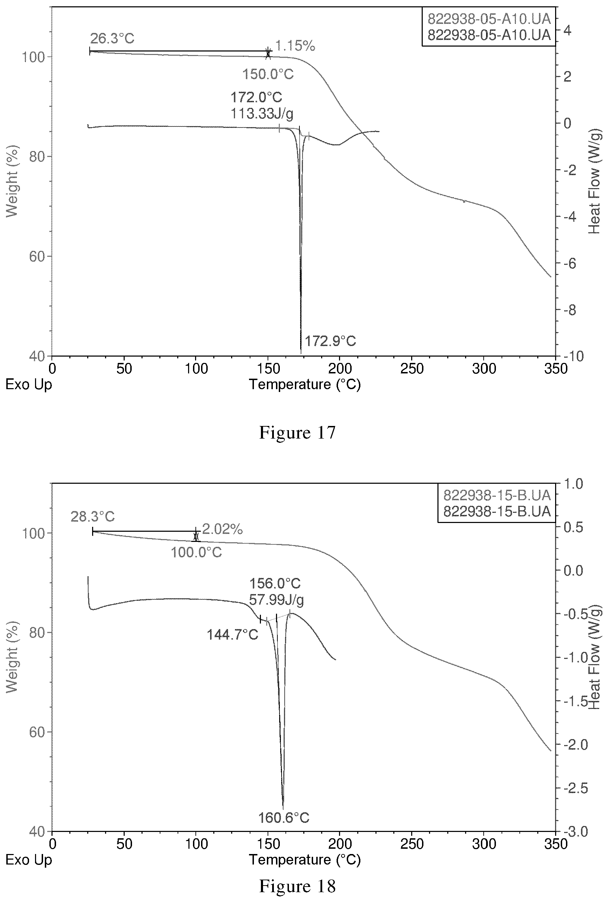

In another preferred embodiment, the succinate crystal form E includes one or more of the following characteristics:
The DSC curve of the succinate crystal form E having an endothermic peak at 172.9±5°C (preferably ±4°C, ±3°C, ±2°C, or ±1°C);
The DSC curve of the succinate crystal form E substantially as shown in Figure 17;
The TGA curve of the succinate crystal form E showing a weight loss of about 1.2±0.2% (preferably ±0.15%, ±0.1%, ±0.05%, or ±0.02%) at 150°C;
The TGA curve of the succinate crystal form E substantially as shown in Figure 17.

In another preferred embodiment, the succinate crystal form E is composed of *N*-(2-(furan-2-yl)-4-((methylamino)methyl)phenyl)thiophene-3-sulfonamide and succinic acid in a molar ratio of 1-1.5:1-1.5, preferably 1-1.2:1-1.2, and more preferably 1:1.

In another preferred embodiment, the solid form is malate crystal form F, wherein the XRPD pattern of the malate crystal form F has characteristic peaks at the following 2θ values: 12.70±0.2°, 14.57±0.2°, 19.13±0.2°, and 19.47±0.2°.

In another preferred embodiment, the malate crystal form F has characteristic peaks at the following 2θ values: 12.70±0.2°, 14.57±0.2°, 18.39±0.2°, 19.13±0.2°, 19.47±0.2°, 22.94±0.2°, 23.77±0.2°, and 24.35±0.2°.

In another preferred embodiment, the malate crystal form F has characteristic peaks at the following 2θ values: 11.41±0.2°, 12.11±0.2°, 12.70±0.2°, 14.57±0.2°, 17.73±0.2°, 18.39±0.2°, 19.13±0.2°, 19.47±0.2°, 22.17±0.2°, 22.49±0.2°, 22.94±0.2°, 23.77±0.2°, 24.35±0.2°, 25.56±0.2°, 26.44±0.2°, 27.48±0.2°, and 28.08±0.2°.

In another preferred embodiment, the X-ray powder diffraction pattern of the malate crystal form F has the following characteristic peaks and peak intensities with 2θ values as shown below:

| 2θ | d value | Relative intensity % |
|---|---|---|
| 11.4110 | 7.75 | 13.78 |
| 12.1109 | 7.31 | 16.79 |
| 12.6952 | 6.97 | 45.24 |
| 14.5657 | 6.08 | 99.82 |
| 15.4788 | 5.72 | 5.87 |
| 17.7330 | 5.00 | 9.05 |
| 18.3939 | 4.82 | 27.67 |
| 19.1258 | 4.64 | 100.00 |
| 19.4667 | 4.56 | 64.62 |
| 20.8204 | 4.27 | 2.82 |
| 21.4947 | 4.13 | 4.20 |
| 22.1669 | 4.01 | 9.76 |
| 22.4900 | 3.95 | 14.16 |
| 22.9372 | 3.88 | 42.20 |
| 23.7676 | 3.74 | 34.35 |
| 24.3523 | 3.66 | 28.65 |
| 25.5641 | 3.48 | 14.56 |
| 26.4387 | 3.37 | 18.92 |
| 26.8956 | 3.32 | 5.67 |
| 27.4865 | 3.25 | 7.78 |
| 28.0766 | 3.18 | 8.03 |
| 28.6498 | 3.12 | 3.90 |
| 29.3684 | 3.04 | 3.81 |
| 30.3043 | 2.95 | 3.30 |
| 31.3934 | 2.85 | 2.83 |
| 32.2886 | 2.77 | 2.58 |
| 32.9310 | 2.72 | 3.29 |
| 33.2893 | 2.69 | 2.07 |
| 34.7057 | 2.58 | 5.70 |
| 37.2071 | 2.42 | 5.47 |
| 38.7947 | 2.32 | 2.11. |

In another preferred embodiment, the malate crystal form F has an XRPD pattern substantially as shown in Figure 6.

In another preferred embodiment, the malate crystal form F includes one or more of the following characteristics:
The DSC curve of the malate crystal form F having endothermic peak at 144.7±5°C (preferably ±4°C, ±3°C, ±2°C, or ±1°C) and 160.6±5°C (preferably ±4°C, ±3°C, ±2°C, or ±1°C);
The DSC curve of the malate crystal form F substantially as shown in Figure 18;
The TGA curve of the malate crystal form F showing a weight loss of about 2.0±0.3% (preferably ±0.2%, ±0.15%, ±0.1%, or ±0.05%) at 100°C;
The TGA curve of the malate crystal form F substantially as shown in Figure 18.

In another preferred embodiment, the malate crystal form F is composed of *N*-(2-(furan-2-yl)-4-((methylamino)methyl)phenyl)thiophene-3-sulfonamide and malic acid in a molar ratio of 1-1.5:1-1.5, preferably 1-1.2:1-1.2, and more preferably 1:1.

In another preferred embodiment, the solid form is phosphate crystal form G, wherein the XRPD pattern of the phosphate crystal form G has characteristic peaks at the following 2θ values: 11.20±0.2°, 19.70±0.2°, 21.24±0.2°, and 22.49±0.2°.

In another preferred embodiment, the phosphate crystal form G has characteristic peaks at the following 2θ values: 11.20±0.2°, 12.79±0.2°, 19.70±0.2°, 21.24±0.2°, 22.49±0.2°, and 23.32±0.2°.

In another preferred embodiment, the phosphate crystal form G has characteristic peaks at the following 2θ values: 11.20±0.2° and 12.79±0.2°, 19.70±0.2°, 20.37±0.2°, 21.24±0.2°, 22.49±0.2°, 23.32±0.2°, 24.64±0.2°, and 30.11±0.2°.

In another preferred embodiment, the X-ray powder diffraction pattern of the phosphate crystal form G has the following characteristic peaks and peak intensities with 2θ values as shown below:

| 2θ | d value | Relative intensity % |
|---|---|---|
| 7.4599 | 11.85 | 5.86 |
| 9.6600 | 9.16 | 4.13 |
| 11.2049 | 7.90 | 100.00 |
| 12.7911 | 6.92 | 22.27 |
| 13.6544 | 6.49 | 3.83 |
| 14.5933 | 6.07 | 7.77 |
| 16.4246 | 5.40 | 6.15 |
| 17.3841 | 5.10 | 1.83 |
| 18.3410 | 4.84 | 2.68 |
| 19.7030 | 4.51 | 52.98 |
| 20.3743 | 4.36 | 10.64 |
| 21.2387 | 4.18 | 40.74 |
| 22.4860 | 3.95 | 49.99 |
| 22.9114 | 3.88 | 7.63 |
| 23.3249 | 3.81 | 23.76 |
| 24.0169 | 3.71 | 3.10 |
| 24.6385 | 3.61 | 10.73 |
| 25.0592 | 3.55 | 7.00 |
| 25.3195 | 3.52 | 6.97 |
| 26.0165 | 3.42 | 4.74 |
| 26.9078 | 3.31 | 3.03 |
| 28.1578 | 3.17 | 9.34 |
| 29.0480 | 3.07 | 1.79 |
| 30.1118 | 2.97 | 17.84 |
| 30.7852 | 2.90 | 3.22 |
| 31.7979 | 2.81 | 7.97 |
| 34.6909 | 2.59 | 2.08 |
| 37.9228 | 2.37 | 2.82. |

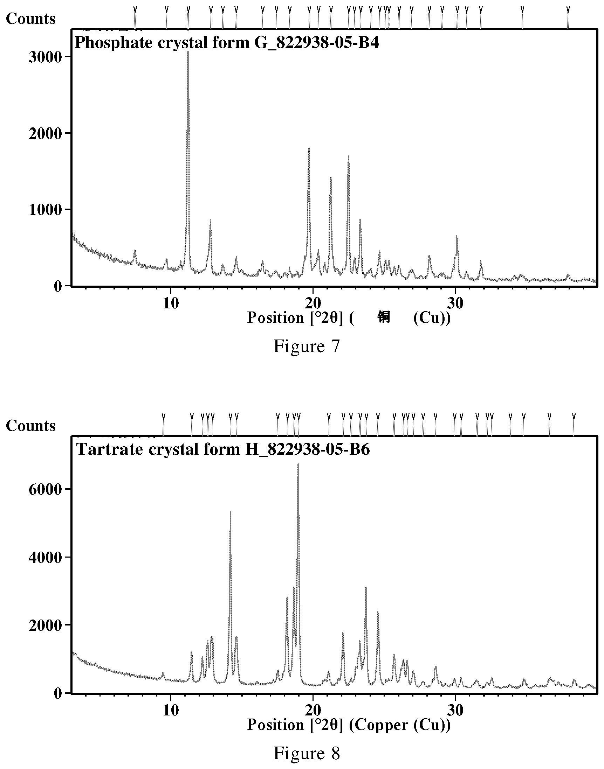

In another preferred embodiment, the phosphate crystal form G has an XRPD pattern substantially as shown in Figure 7.

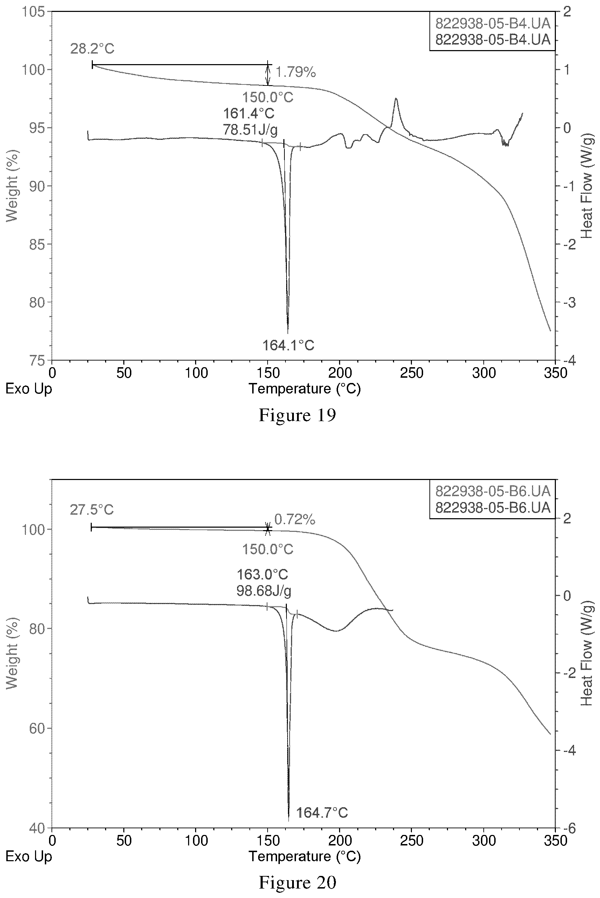

In another preferred embodiment, the phosphate crystal form G includes one or more of the following characteristics:
The DSC curve of the phosphate crystal form G having an endothermic peak at 164.1±5°C (preferably ±4°C, ±3°C, ±2°C, or ±1°C);
The DSC curve of the phosphate crystal form G substantially as shown in Figure 19;
The TGA curve of the phosphate crystal form G showing a weight loss of about 1.8% (preferably ±0.2%, ±0.15%, ±0.1%, or ±0.05%) at 150°C;
The TGA curve of the phosphate crystal form G substantially as shown in Figure 19.

In another preferred embodiment, the phosphate crystal form G is composed of *N*-(2-(furan-2-yl)-4-((methylamino)methyl)phenyl)thiophene-3-sulfonamide and phosphoric acid in a molar ratio of 1-1.5:1-1.5, preferably 1-1.2:1-1.2, and more preferably 1:1.

In another preferred embodiment, the solid form is tartrate crystal form H, wherein the XRPD pattern of the tartrate crystal form H has characteristic peaks at the following 2θ values: 14.19±0.2°, 18.64±0.2°, 18.95±0.2°, and 23.70±0.2°.

In another preferred embodiment, the tartrate crystal form H has characteristic peaks at the following 2θ values: 14.19±0.2°, 18.17±0.2°, 18.64±0.2°, 18.95±0.2°, 22.11±0.2°, 23.70±0.2°, and 24.54±0.2°.

In another preferred embodiment, the tartrate crystal form H has characteristic peaks at the following 2θ values: 11.45±0.2°, 12.57±0.2°, 12.92±0.2°, 14.19±0.2°, 14.62±0.2°, 18.17±0.2°, 18.64±0.2°, 18.95±0.2°, 22.11±0.2°, 23.30±0.2°, 23.70±0.2°, 24.54±0.2°, and 25.68±0.2°.

In another preferred embodiment, the X-ray powder diffraction pattern of the tartrate crystal form H has the following characteristic peaks and peak intensities with 2θ values as shown below:

| 2θ | d value | Relative intensity % |
|---|---|---|
| 9.4501 | 9.36 | 3.00 |
| 11.4519 | 7.73 | 12.58 |
| 12.2140 | 7.25 | 10.34 |
| 12.5744 | 7.04 | 16.86 |
| 12.9151 | 6.85 | 18.46 |
| 14.1904 | 6.24 | 69.10 |
| 14.6289 | 6.06 | 18.37 |
| 17.5049 | 5.07 | 5.74 |
| 18.1662 | 4.88 | 35.82 |
| 18.6402 | 4.76 | 39.64 |
| 18.9495 | 4.68 | 100.00 |
| 21.1009 | 4.21 | 5.21 |
| 22.1089 | 4.02 | 20.99 |
| 22.6438 | 3.93 | 2.77 |
| 23.3031 | 3.82 | 17.75 |
| 23.7026 | 3.75 | 39.96 |
| 24.5396 | 3.63 | 30.08 |
| 25.6815 | 3.47 | 12.75 |
| 26.3527 | 3.38 | 10.20 |
| 26.6114 | 3.35 | 9.14 |
| 27.0443 | 3.30 | 6.23 |
| 27.7255 | 3.22 | 1.92 |
| 28.6003 | 3.12 | 7.72 |
| 29.9503 | 2.98 | 3.52 |
| 30.3960 | 2.94 | 3.88 |
| 31.5252 | 2.84 | 2.91 |
| 32.2115 | 2.78 | 2.18 |
| 32.5255 | 2.75 | 4.12 |
| 33.8337 | 2.65 | 1.02 |
| 34.7938 | 2.58 | 3.98 |
| 36.6121 | 2.45 | 3.29 |
| 38.3299 | 2.35 | 3.41. |

In another preferred embodiment, the tartrate crystal form H has an XRPD pattern substantially as shown in Figure 8.

In another preferred embodiment, the tartrate crystal form H includes one or more of the following characteristics:
The DSC curve of the tartrate crystal form H having an endothermic peak at 164.7±5°C (preferably ±4°C, ±3°C, ±2°C, or ±1°C);
The DSC curve of the tartrate crystal form H substantially as shown in Figure 20;
The TGA curve of the tartrate crystal form H showing a weight loss of approximately 0.72% (preferably ±0.1%, ±0.05%, ±0.02%, or ±0.01%) at 150°C;
The TGA curve of the tartrate crystal form H substantially as shown in Figure 20.

In another preferred embodiment, the tartrate crystal form H is composed of *N*-(2-(furan-2-yl)-4-((methylamino)methyl)phenyl)thiophene-3-sulfonamide and tartaric acid in a molar ratio of 1-1.5:1-1.5, preferably 1-1.2:1-1.2, and more preferably 1:1.

In another preferred embodiment, the solid form is pyroglutamate crystal form I, wherein the XRPD pattern of the pyroglutamate crystal form I has characteristic peaks at the following 2θ values: 9.29±0.2°, 10.76±0.2°, 17.98±0.2°, and 23.72±0.2°.

In another preferred embodiment, the pyroglutamate crystal form I has characteristic peaks at the following 2θ values: 9.29±0.2°, 10.76±0.2°, 17.98±0.2°, 19.93±0.2°, 21.60±0.2°, 21.86±0.2°, and 23.72±0.2°.

In another preferred embodiment, the pyroglutamate crystal form I has characteristic peaks at the following 2θ values: 9.29±0.2°, 10.76±0.2°, 14.33±0.2°, 16.91±0.2°, 17.81±0.2°, 17.98±0.2°, 18.83±0.2°, 19.93±0.2°, 21.60±0.2°, 21.86±0.2°, 22.98±0.2°, 23.72±0.2°, and 30.86±0.2°.

In another preferred embodiment, the X-ray powder diffraction pattern of the pyroglutamate crystal form I has the following characteristic peaks and peak intensities with 2θ values as shown below:

| 2θ | d value | Relative intensity % |
|---|---|---|
| 9.2936 | 9.52 | 61.85 |
| 10.7641 | 8.22 | 100.00 |
| 12.8197 | 6.91 | 1.19 |
| 14.3250 | 6.18 | 23.11 |
| 15.6481 | 5.66 | 8.01 |
| 16.9138 | 5.24 | 15.79 |
| 17.5344 | 5.06 | 11.11 |
| 17.8066 | 4.98 | 49.54 |
| 17.9827 | 4.93 | 85.60 |
| 18.8311 | 4.71 | 16.60 |
| 19.3811 | 4.58 | 12.20 |
| 19.9315 | 4.45 | 42.18 |
| 20.9393 | 4.24 | 8.97 |
| 21.2892 | 4.17 | 13.95 |
| 21.6021 | 4.11 | 30.47 |
| 21.8585 | 4.07 | 33.68 |
| 22.9760 | 3.87 | 28.93 |
| 23.7199 | 3.75 | 51.56 |
| 24.4718 | 3.64 | 3.40 |
| 25.2288 | 3.53 | 2.51 |
| 26.2478 | 3.40 | 10.02 |
| 26.7024 | 3.34 | 6.18 |
| 27.7420 | 3.22 | 7.20 |
| 28.2762 | 3.16 | 11.72 |
| 29.5520 | 3.02 | 2.41 |
| 30.4589 | 2.93 | 3.38 |
| 30.8590 | 2.90 | 23.21 |
| 31.5895 | 2.83 | 2.59 |
| 31.9561 | 2.80 | 5.22 |
| 32.9723 | 2.72 | 10.86 |
| 34.6644 | 2.59 | 2.52 |
| 35.5174 | 2.53 | 5.68 |
| 36.6386 | 2.45 | 1.53 |
| 37.4019 | 2.40 | 1.88 |
| 38.6224 | 2.33 | 4.00. |

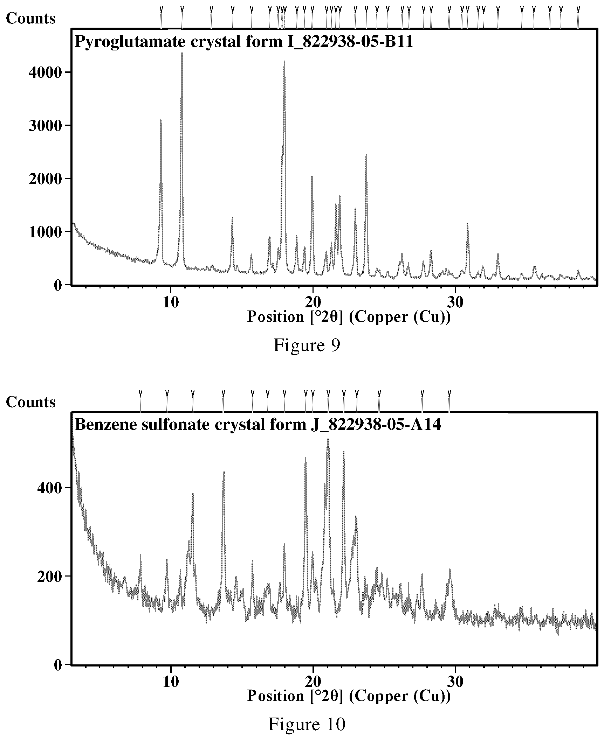

In another preferred embodiment, the pyroglutamate crystal form I has an XRPD pattern substantially as shown in Figure 9.

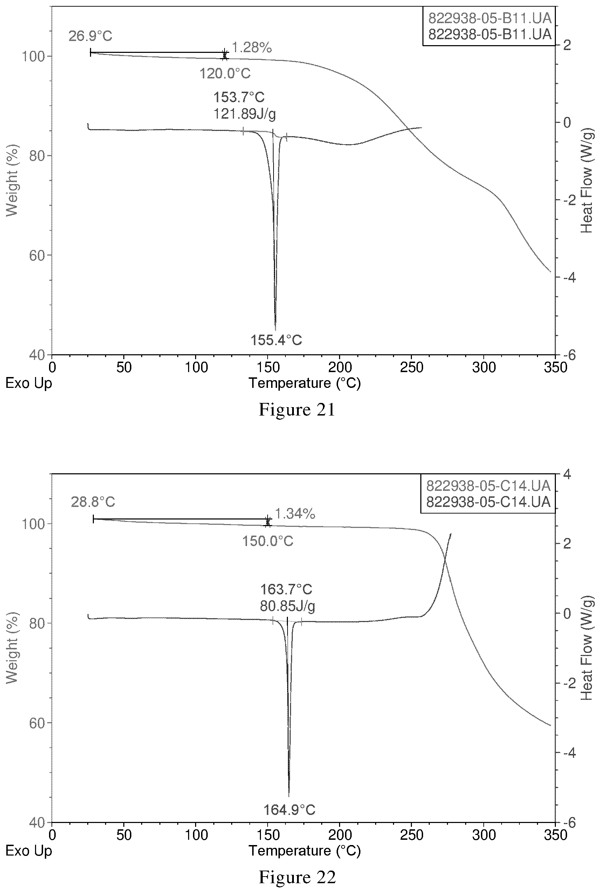

In another preferred embodiment, the pyroglutamate crystal form I includes one or more of the following characteristics:
The DSC curve of the pyroglutamate crystal form I having an endothermic peak at 155.4±5°C (preferably ±4°C, ±3°C, ±2°C, or ±1°C);
The DSC curve of the pyroglutamate crystal form I substantially as shown in Figure 21;
The TGA curve of the pyroglutamate crystal form I showing 1.3% weight loss (preferably ±02%, ±0.15%, ±0.1%, or ±0.05%) at 120°C;
The TGA curve of the pyroglutamate crystal form I substantially as shown in Figure 21.

In another preferred embodiment, the pyroglutamate crystal form I is composed of *N*-(2-(furan-2-yl)-4-((methylamino)methyl)phenyl)thiophene-3-sulfonamide and pyroglutamic acid in a molar ratio of 1-1.5:1-1.5, preferably 1-1.2:1-1.2, and more preferably 1:1.

In another preferred embodiment, the solid form is benzene sulfonate crystal form J, and the XRPD pattern of the benzene sulfonate crystal form J has characteristic peaks at the following 2θ values: 13.69±0.2°, 19.48±0.2°, 21.07±0.2°, and 22.15±0.2°.

In another preferred embodiment, the benzene sulfonate crystal form J has characteristic peaks at the following 2θ values: 11.53±0.2°, 13.69±0.2°, 17.96±0.2°, 19.48±0.2°, 21.07±0.2°, 22.15±0.2°, and 23.06±0.2°.

In another preferred embodiment, the benzene sulfonate crystal form J has characteristic peaks at the following 2θ values: 9.70±0.2°, 11.53±0.2°, 13.69±0.2°, 15.72±0.2°, 17.96±0.2°, 19.48±0.2°, 19.96±0.2°, 21.07±0.2°, 22.15±0.2°, 23.06±0.2°, 27.64±0.2°, and 29.58±0.2°.

In another preferred embodiment, the X-ray powder diffraction pattern of the benzene sulfonate crystal form J has the following characteristic peaks and peak intensities with 2θ values as shown below:

| 2θ | d value | Relative intensity % |
|---|---|---|
| 7.8263 | 11.30 | 18.85 |
| 9.7005 | 9.12 | 23.70 |
| 11.5344 | 7.67 | 63.01 |
| 13.6926 | 6.47 | 72.52 |
| 15.7215 | 5.64 | 24.00 |
| 16.7789 | 5.28 | 14.03 |
| 17.9617 | 4.94 | 37.91 |
| 19.4764 | 4.56 | 82.26 |
| 19.9603 | 4.45 | 33.29 |
| 21.0708 | 4.22 | 100.00 |
| 22.1505 | 4.01 | 85.15 |
| 23.0614 | 3.86 | 48.53 |
| 24.6362 | 3.61 | 15.71 |
| 27.6427 | 3.23 | 21.08 |
| 29.5771 | 3.02 | 22.37. |

In another preferred embodiment, the benzene sulfonate crystal form J has an XRPD pattern substantially as shown in Figure 10.

In another preferred embodiment, the benzene sulfonate crystal form J includes one or more of the following characteristics:
The DSC curve of the benzene sulfonate crystal form J having an endothermic peak at 164.9±5°C (preferably ±4°C, ±3°C, ±2°C, or ±1°C);
The DSC curve of the benzene sulfonate crystal form J substantially as shown in Figure 22;
The TGA curve of the benzene sulfonate crystal form J showing a weight loss of about 1.3% (preferably ±0.2%, ±0.15%, ±0.1%, or ±0.05%) at 150°C;
The TGA curve of the benzene sulfonate crystal form J substantially as shown in Figure 22.

In another preferred embodiment, the benzene sulfonate crystal form J is composed of *N*-(2-(furan-2-yl)-4-((methylamino)methyl)-phenyl)thiophene-3-sulfonamide and benzene sulfonic acid in a molar ratio of 1-1.5:1-1.5, preferably 1-1.2:1-1.2, and more preferably 1:1.

In another preferred embodiment, the solid form is malonate crystal form K, wherein the XRPD pattern of the malonate crystal form K has characteristic peaks at the following 2θ values: 15.28±0.2°, 19.66±0.2°, and 20.42±0.2°.

In another preferred embodiment, the malonate crystal form K has characteristic peaks at the following 2θ values: 15.28±0.2°, 19.43±0.2°, 19.66±0.2°, 20.42±0.2°, and 23.66±0.2°.

In another preferred embodiment, the malonate crystal form K has characteristic peaks at the following 2θ values: 13.71±0.2°, 15.28±0.2°, 19.43±0.2°, 19.66±0.2°, 20.42±0.2°, 22.66±0.2°, 23.66±0.2°, 25.46±0.2°, 26.42±0.2°, and 27.82±0.2°.

In another preferred embodiment, the X-ray powder diffraction pattern of the malonate crystal form K has the following characteristic peaks and peak intensities with 2θ values as shown below:

| 2θ | d value | Relative intensity % |
|---|---|---|
| 11.7720 | 7.52 | 11.86 |
| 12.6542 | 7.00 | 12.02 |
| 13.7132 | 6.46 | 13.76 |
| 15.2846 | 5.80 | 100.00 |
| 15.8543 | 5.59 | 7.35 |
| 16.3506 | 5.42 | 3.99 |
| 18.9714 | 4.68 | 10.90 |
| 19.4289 | 4.57 | 21.60 |
| 19.6560 | 4.52 | 24.43 |
| 20.4179 | 4.35 | 38.71 |
| 22.6553 | 3.92 | 13.46 |
| 23.6581 | 3.76 | 20.02 |
| 24.3848 | 3.65 | 12.06 |
| 25.4604 | 3.50 | 17.25 |
| 26.4165 | 3.37 | 18.93 |
| 27.2939 | 3.27 | 4.65 |
| 27.8164 | 3.21 | 13.05 |
| 30.8357 | 2.90 | 2.01. |

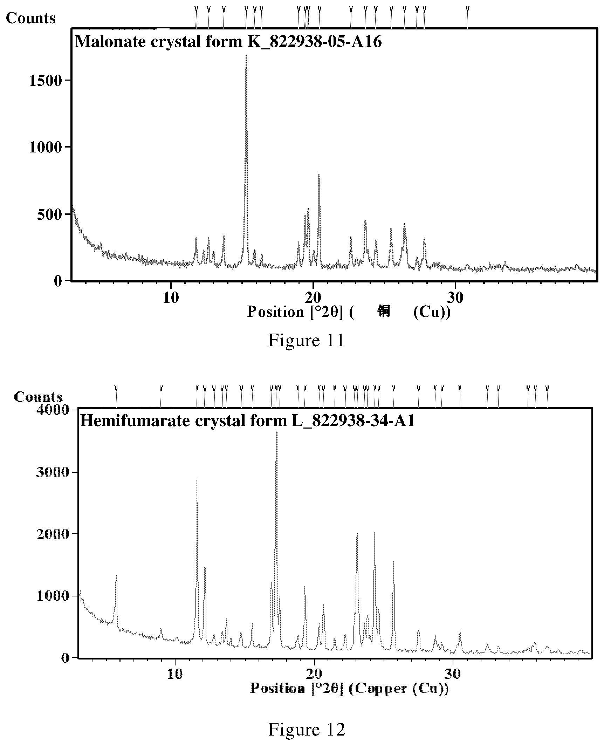

In another preferred embodiment, the malonate crystal form K has an XRPD pattern substantially as shown in Figure 11.

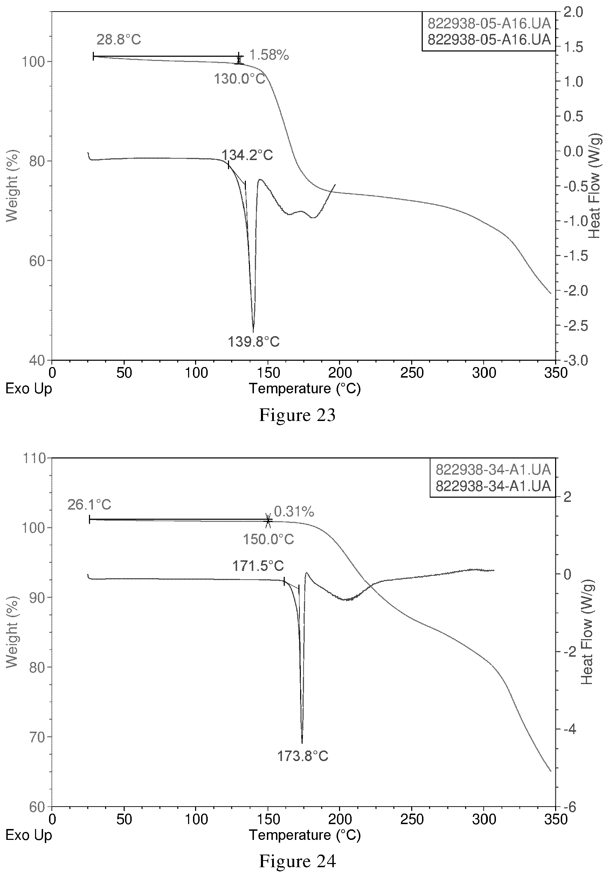

In another preferred embodiment, the malonate crystal form K includes one or more of the following characteristics:
The DSC curve of the malonate crystal form K having an endothermic peak at 139.8±5°C (preferably ±4°C, ±3°C, ±2°C, or ±1°C);
The DSC curve of the malonate crystal form K substantially as shown in Figure 23;
The TGA curve of the malonate crystal form K showing a weight loss of 1.58±0.2% (preferably ±0.15%, ±0.1%, ±0.05%, or ±0.02%) at 130°C;
The TGA curve of the malonate crystal form K substantially as shown in Figure 23.

In another preferred embodiment, the malonate crystal form K is composed of *N*-(2-(furan-2-yl)-4-((methylamino)methyl)phenyl) thiophene-3-sulfonamide and malonic acid in a molar ratio of 1-1.5:1-1.5, preferably 1-1.2:1-1.2, and more preferably 1:1.

In another preferred embodiment, the solid form is hemifumarate crystal form L, and the XRPD pattern of the hemifumarate crystal form L has characteristic peaks at the following 2θ values: 11.57±0.2°, 17.25±0.2°, 23.08±0.2°, and 24.33±0.2°.

In another preferred embodiment, the hemifumarate crystal form L has characteristic peaks at the following 2θ values: 11.57±0.2°, 12.12±0.2°, 17.25±0.2°, 23.08±0.2°, 24.33±0.2°, and 25.68±0.2°.

In another preferred embodiment, the hemifumarate crystal form L has characteristic peaks at the following 2θ values: 5.75±0.2°, 11.57±0.2°, 12.12±0.2°, 16.91±0.2°, 17.25±0.2°, 17.50±0.2°, 19.31±0.2°, 20.67±0.2°, 23.08±0.2°, 24.33±0.2°, and 25.68±0.2°.

In another preferred embodiment, the X-ray powder diffraction pattern of the hemifumarate crystal form L has the following characteristic peaks and peak intensities with 2θ values as shown below:

| 2θ | d value | Relative intensity % |
|---|---|---|
| 5.7467 | 15.38 | 21.65 |
| 8.9553 | 9.87 | 4.23 |
| 11.5652 | 7.65 | 67.24 |
| 12.1205 | 7.30 | 31.74 |
| 12.7752 | 6.93 | 4.53 |
| 13.3790 | 6.62 | 5.53 |
| 13.6634 | 6.48 | 11.42 |
| 14.7284 | 6.01 | 5.86 |
| 15.5191 | 5.71 | 10.25 |
| 16.9116 | 5.24 | 27.22 |
| 17.2506 | 5.14 | 100.00 |
| 17.5018 | 5.07 | 21.59 |
| 18.8042 | 4.72 | 5.64 |
| 19.3060 | 4.60 | 25.92 |
| 20.3231 | 4.37 | 10.70 |
| 20.6655 | 4.30 | 18.63 |
| 21.4639 | 4.14 | 4.44 |
| 22.2232 | 4.00 | 6.22 |
| 22.8905 | 3.89 | 14.42 |
| 23.0787 | 3.85 | 47.91 |
| 23.5842 | 3.77 | 11.85 |
| 23.8077 | 3.74 | 14.53 |
| 24.3257 | 3.66 | 48.70 |
| 24.6192 | 3.62 | 16.58 |
| 25.6766 | 3.47 | 37.11 |
| 27.5081 | 3.24 | 8.66 |
| 28.7002 | 3.11 | 7.19 |
| 29.1791 | 3.06 | 3.76 |
| 30.4714 | 2.93 | 9.77 |
| 32.4509 | 2.76 | 3.66 |
| 33.2324 | 2.70 | 2.78 |
| 35.3682 | 2.54 | 2.19 |
| 35.9013 | 2.50 | 4.06 |
| 36.7589 | 2.45 | 2.58. |

In another preferred embodiment, the hemifumarate crystal form L has an XRPD pattern substantially as shown in Figure 12.

In another preferred embodiment, the hemifumarate crystal form L includes one or more of the following characteristics:
The DSC curve of the hemifumarate crystal form L having an endothermic peak at 171.5±5°C (preferably ±4°C, ±3°C, ±2°C, or ±1°C);
The DSC curve of the hemifumarate crystal form L substantially as shown in Figure 24;
The TGA curve of the hemifumarate crystal form L showing 0.3% weight loss (preferably ±0.1%,or 0.05% ) at 150±2°C;
The TGA curve of the hemifumarate crystal form L substantially as shown in Figure 24.

In another preferred embodiment, the hemifumarate crystal form L is composed of *N*-(2-(furan-2-yl)-4-((methylamino)methyl)-phenyl)thiophene-3-sulfonamide and fumaric acid in a molar ratio of 1-1.5:0.5-0.75, preferably 1-1.2:0.5-0.6, and more preferably 1:0.5.

In the second aspect, the present invention provides a method for preparing the solid form of *N*-(2-(furan-2-yl)-4-((methylamino)methyl)phenyl)thiophene-3-sulfonamide as described in the first aspect, wherein the method includes any one of Method One to Eight:
Method One comprises:
(a1) dissolving the prepared crude compound in a first solvent, heating, and mixing until clear;
(a2) adding a second solvent, cooling and crystallizing to obtain the free crystal form A;

In another preferred embodiment, in Method One, the crude compound is obtained by filtration, washing and vacuum drying according to the method described in Chinese Patent Application No. 202110666168.6.

In another preferred embodiment, the first solvent in step (a1) is selected from the group consisting of dichloromethane, methyl tert-butyl ether, toluene, tetrahydrofuran, dimethyl sulfoxide, and combinations thereof. Preferably the first solvent is dimethyl sulfoxide.

In another preferred embodiment, the volume ratio of the first solvent to the crude compound in step (a1) is 1.5-3:1, preferably 2:1.

In another preferred embodiment, the heating temperature in step (a1) refers to heating to 50-70°C, preferably 60-70°C.

In another preferred embodiment, the mixing time in step (a1) is 0.3-1 h, preferably 0.5 h.

In another preferred embodiment, the second solvent in step (a2) is selected from the group consisting of methanol, absolute ethanol, 95% ethanol, ethanol/water, acetone/water, acetonitrile/water, or combinations thereof. Preferably the second solvent is methanol, absolute ethanol or 95% ethanol. More preferably the second solvent is absolute ethanol.

In another preferred embodiment, the volume ratio of the second solvent to the crude compound in step (a2) is 1.5-5:1, for example 2:1 or 3:1.

In another preferred embodiment, the second solvent in step (a2) is added at 50-70°C, preferably 60-70°C.

In another preferred embodiment, the cooling crystallization in step (a2) includes cooling crystallization step by step.

In another preferred embodiment, the cooling crystallization in step (a2) comprises: after stirring for 0.5 hours, turning off heating, slowly cooling to 0-10°C, and maintaining at 0-10°C while stirring for 4 hours.

Method Two comprises: dissolving and mixing compound raw material and fumaric acid in a third solvent, cooling and crystallizing to obtain the fumarate crystal form B.

In another preferred embodiment, the third solvent is selected from the group consisting of: methanol, absolute ethanol, 95% ethanol, ethanol/water, acetone/water, acetonitrile/water, ethyl acetate, acetone, or combinations thereof. Preferably the third solvent is methanol or 95% ethanol, more preferably methanol.

In another preferred embodiment, the molar ratio of the compound raw material to fumaric acid in Method Two is 1:1-1.5, preferably 1:1-1.2, such as 1:1.02, 1:1.04, 1:1.06, 1:1.1, 1:1.12, 1:1.15, or 1:1.2.

In another preferred embodiment, the mass-to-volume ratio of the compound raw material to third solvent in Method Two is 1g:1-20ml, preferably 1g:1-10ml, such as 1g:3ml, 1g:6ml or 1g:9ml.

In another preferred embodiment, the mixing in Method Two is conducted at 0-15°C, preferably 3-10°C, and more preferably 4-6°C.

In another preferred embodiment, the mixing time in Method Two is 0.5-2 days, preferably 1 day.

In another preferred embodiment, Method Two comprises dissolving the compound raw material and fumaric acid in the third solvent, and stirring at 5°C for 1 day.

In another preferred embodiment, Method Two further comprises post-treatment steps of suction filtration and vacuum drying.

Method Three comprises:
(b1) dissolving compound raw material in a fourth solvent;
(b2) dissolving concentrated hydrochloric acid in a fourth solvent;
(b3) adding the solution of hydrochloric acid in the fourth solvent obtained in step (b2) dropwise into the mixture obtained in step (b1), mixing and crystallizing to obtain the hydrochloride crystal form C.

In another preferred embodiment, the **fourth solvent in step (b1) is selected from the group consisting of: dichloromethane, methyl tert-butyl ether, toluene, tetrahydrofuran, or combinations thereof. Preferably the fourth solvent is tetrahydrofuran.**

In another preferred embodiment, **the concentrated hydrochloric acid is an aqueous HCl solution with a mass percentage of 35-40%.**

In another preferred embodiment, **the molar ratio of compound raw material to the concentrated hydrochloric acid in Method** Three **is 1:1-1.5, preferably 1:1-1.4, such as 1:1.02, 1:1.04, 1:1.06, 1:1.1, 1:1.12, 1:1.15, 1:1.2, or 1:1.4.**

In another preferred embodiment, **the volume ratio of the fourth solvent used in step (b1) to that used in step (b2) is 1-1.5:1-1.5, preferably 1-1.2:1-1.2, and more preferably 1:1.**

In another preferred embodiment, **the mixing in step (b3) is conducted at 10-35°C.**

In another preferred embodiment, **the mixing time in step (b3) is 0.5-2 h, preferably 1 h.**

In another preferred embodiment, **the step (b3) comprises slowly adding the solution of hydrochloric acid in the fourth solvent into the suspension of compound raw material under stirring, followed by stirring at room temperature for 1 hour.**

In another preferred embodiment, **Method** Three **further comprises post-treatment steps of suction filtration and vacuum drying.**

Method Four comprises:
(c1) dissolving compound raw material in a fifth solvent;
(c2) dissolving concentrated sulfuric acid in a fifth solvent;
(c3) adding the solution of sulfuric acid in the fifth solvent obtained in step (c2) dropwise into the mixture obtained in step (c1), and dissolving to clear;
(c4) adding a sixth solvent dropwise into the mixture obtained in step (c3), mixing and crystallizing to obtain the sulfate crystal form D.

In another preferred embodiment, **the fifth solvent in step (c1) is a mixed solvent of organic solvent and water, which is selected from the group consisting of: ethanol/water, acetone/water, acetonitrile/water, or combinations thereof. Preferably the fifth solvent is a mixed solvent of acetonitrile/water.**

In another preferred embodiment, **the volume ratio of the organic solvent to water in the fifth solvent in step (c1) is 10-30:1, such as 15:1, 19:1, 20:1, 25:1, or 30:1.**

In another preferred embodiment, **the concentrated sulfuric acid in step (c2) is a 95-98 wt% aqueous H₂SO₄ solution.**

In another preferred embodiment, **the molar ratio of compound raw material to the concentrated sulfuric acid in Method** Four **is 1:1-1.5, such as 1:1.1, 1:1.15, 1:1.2, 1:3, or 1:4.**

In another preferred embodiment, **the volume ratio of compound raw material to the total amount of fifth solvent used in Method** Four **is 1g:10-40ml, preferably 1g:10-30ml, such as 1g:15ml, 1g:20ml, 1g:25ml, or 1g:30ml.**

In another preferred embodiment, **the volume ratio of the fifth solvent used in step (c1) to that used in step (c2) is 1-1.5:1-1.5, preferably 1-1.2:1-1.2, more preferably 1:1.**

In another preferred embodiment, **the step (c3) comprises slowly adding the solution of sulfuric acid in the fifth solvent into the suspension of the free form sample under stirring until complete dissolution.**

In another preferred embodiment, **the sixth solvent in step (c4) is selected from the group consisting of: dichloromethane, methyl tert-butyl ether, toluene, tetrahydrofuran, or combinations thereof. Preferably the sixth solvent is methyl tert-butyl ether.**

In another preferred embodiment, **the volume ratio of the total amount of fifth solvent used in Method Four to the sixth solvent is 1:1.5-3, such as 1:1.7, 1:2, 1:2.5, or 1:3.**

In another preferred embodiment, **the mixing in step (c4) is conducted at 10-35°C.**

In another preferred embodiment, **the mixing time in step (c4) is 0.5-2 h, preferably 1 h.**

In another preferred embodiment, **the step (c4) comprises slowly adding the sixth solvent into the system under stirring, and stirring at room temperature for 1 hour to crystallize.**

In another preferred embodiment, **Method** Four **further comprises post-treatment steps of suction filtration and vacuum drying.**

Method Five comprises:
dissolving compound raw material and succinic acid in a seventh solvent, mixing and crystallizing to obtain the succinate crystal form E.

In another preferred embodiment, the seventh solvent is selected from the group consisting of: methanol, absolute ethanol, 95% ethanol, ethanol/water, acetone/water, acetonitrile/water, ethyl acetate, acetone, or combinations thereof. Preferably the seventh solvent is methanol or 95% ethanol. More the seventh solvent is preferably methanol.

In another preferred embodiment, the molar ratio of compound raw material to succinic acid in Method Five is 1:1-1.5, preferably 1:1-1.2, such as 1:1.02, 1:1.04, 1:1.06, 1:1.1, 1:1.12, 1:1.15, or 1:1.2.

In another preferred embodiment, the mass-to-volume ratio of compound raw material to the seventh solvent in Method Five is 1g:1-20mL, preferably 1g:1-10mL, such as 1g:3mL, 1g:6mL, or 1g:9mL.

In another preferred embodiment, the mixing in Method Five is performed at 10-35°C.

In another preferred embodiment, the mixing time in Method Five is 0.5-2 days, preferably 1 day.

In another preferred embodiment, Method Five comprises the steps of: dissolving compound raw material and succinic acid in the seventh solvent, and stirring at room temperature for 1 day.

In another preferred embodiment, Method Five further comprises post-treatment steps of filtration under reduced pressure and vacuum drying.

Method Six comprises:
dissolving compound raw material and malic acid in an eighth solvent, mixing and crystallizing to obtain the malate crystal form F.

**In another preferred embodiment, the eighth solvent is selected from the group consisting of: dichloromethane, methyl tert-butyl ether, toluene, tetrahydrofuran, or a combination thereof. Preferably the eighth solvent is tetrahydrofuran.**

**In another preferred embodiment, the molar ratio of compound raw material to malic acid is 1:1-1.5, preferably 1:1-1.2, such as 1:1.02, 1:1.04, 1:1.06, 1:1.1, 1:1.12, 1:1.15, or 1:1.2.**

**In another preferred embodiment, the mass-to-volume ratio of compound raw material to the eighth solvent is 1 g:1-20 mL, preferably 1 g:1-15 mL, such as 1 g:5 mL, 1 g:9 mL, 1 g:10 mL, or 1 g:12 mL.**

**In another preferred embodiment, the mixing is conducted at a temperature of 10-35°C.**

**In another preferred embodiment, the mixing duration is 0.5-2 days, preferably 1 day.**

**In another preferred embodiment, Method** Six **comprises the steps of: dissolving compound raw material and malic acid in the eighth solvent, and stirring at room temperature for 1 day.**

**In another preferred embodiment, Method** Six **further comprises post-treatment steps of suction filtration and vacuum drying.**

Method Seven comprises:
dissolving compound raw material and an acid in a ninth solvent, mixing and crystallizing to obtain the phosphate crystal form G, the tartrate crystal form H, the pyroglutamate crystal form I, the benzenesulfonate crystal form J, and the malonate crystal form K that correspond to the acid; wherein the acid is selected from the group consisting of phosphoric acid, tartaric acid, pyroglutamic acid, benzenesulfonic acid, or malonic acid.

In another preferred embodiment, the ninth solvent is selected from the group consisting of: dichloromethane, methyl tert-butyl ether, toluene, tetrahydrofuran, or combinations thereof. **Preferably** the ninth solvent is tetrahydrofuran.

In another preferred embodiment, the molar ratio of compound raw material to the acid is 1:1-1.5, preferably 1:1-1.2, such as 1:1.02, 1:1.04, 1:1.06, 1:1.1, 1:1.12, 1:1.15, or 1:1.2.

In another preferred embodiment, the concentration of compound raw material in the ninth solvent is 0.05-0.5 mol/L, preferably 0.05-0.3 mol/L, such as 0.08 mol/L, 0.1 mol/L, 0.12 mol/L, or 0.15 mol/L.

In another preferred embodiment, the mixing is performed at 10-35°C.

In another preferred embodiment, Method Seven further comprises post-treatment steps of filtration under reduced pressure and vacuum drying.

Method Eight comprises:
dissolving compound raw material and fumaric acid in a third solvent, mixing and crystallizing to obtain the hemifumarate crystal form L.

In another preferred embodiment, the third solvent is selected from the group consisting of: methanol, anhydrous ethanol, 95% ethanol, ethanol/water, acetone/water, acetonitrile/water, ethyl acetate, acetone, or a combination thereof. **Preferably** the third solvent is methanol or 95% ethanol. More **preferably** the third solvent is methanol.

In another preferred embodiment, the molar ratio of compound raw material to fumaric acid is 1:0.5-0.75, preferably 1:0.5-0.6, such as 1:0.5, 1:0.55, 1:0.58, or 1:0.6.

In another preferred embodiment, the mass-to-volume ratio of compound raw material to the third solvent is 1 g:1-10 mL, preferably 1 g:1-5 mL, such as 1 g:2 mL, 1 g:3 mL, or 1 g:4 mL.

In another preferred embodiment, the mixing is performed at room temperature, preferably at 10-35°C, more preferably at 15-25°C.

In another preferred embodiment, the mixing duration is 1-7 days, preferably 4 days.

In another preferred embodiment, Method Eight comprises the steps of: dissolving compound raw material and fumaric acid in the third solvent, and suspension stirring at room temperature for 4 days.

In another preferred embodiment, Method Eight further comprises post-treatment steps of filtration under suction and vacuum drying.

In another preferred embodiment, the compound raw material comprises an amorphous form or crystalline form of *N*-(2-(furan-2-yl)-4-((methylamino)methyl)phenyl)thiophene-3-sulfonamide, preferably the free crystal form A.

In the third aspect, the present invention provides a pharmaceutical composition comprising:
(a) one or more solid forms of *N*-(2-(furan-2-yl)-4-((methylamino)methyl)phenyl)thiophene-3-sulfonamide as described in the first aspect of the present invention; and
(b) pharmaceutically acceptable adjuvants, carriers, excipients, or diluents.

In the fourth aspect, the present invention provides a use of the solid form of *N*-(2-(furan-2-yl)-4-((methylamino)methyl)phenyl)thiophene-3-sulfonamide as described in the first aspect of the present invention in (a) preparing an inhibitor of transient receptor potential channel TRPA1; and/or (b) preparing a drug for preventing and/or treating diseases associated with transient receptor potential channel TRPA1.

**In another preferred embodiment, the disease associated with transient receptor potential channel TRPA1 is selected from the group consisting of inflammatory bowel disease, irritable bowel syndrome, pain, inflammation, cough, or combinations thereof.**

**In another preferred embodiment, the inflammatory bowel disease includes Crohn's disease and/or ulcerative colitis.**

In another preferred embodiment, the pain includes visceral pain, acute inflammatory pain, chronic inflammatory pain, neuropathic pain, fibromyalgia, headache, neuralgia, or cancer-associated pain.

In a fifth aspect, the present invention provides a method for (a) inhibiting transient receptor potential channel protein TRPA1; and/or (b) preventing and/or treating diseases related to transient receptor potential channel protein TRPA1, comprising the steps of administering to a subject in need thereof the solid form of *N*-(2-(furan-2-yl)-4-((methylamino)methyl)phenyl)thiophene-3-sulfonamide described in the first aspect of the present invention.

**In another preferred embodiment, the subject is a human or a non-human mammal.**

It should be understood that, within the scope of the present invention, the technical features described above and those specifically described hereinafter (e.g., in the examples) may be mutually combined to form new or preferred technical solutions. Due to space limitations, such combinations are not enumerated here.

### Description of The Drawings

**Figure** 1 is an XRPD pattern of free crystal form A.
**Figure** 2 is an XRPD pattern of fumarate crystal form B.
**Figure** 3 is an XRPD pattern of hydrochloride crystal form C.
**Figure** 4 is an XRPD pattern of sulfate crystal form D.
**Figure** 5 is an XRPD pattern of succinate crystal form E.
**Figure** 6 is an XRPD pattern of malate crystal form F.
**Figure** 7 is an XRPD pattern of phosphate crystal form G.
**Figure** 8 is an XRPD pattern of **tartrate crystal form H.**
**Figure** 9 is an XRPD pattern of pyroglutamate crystal form I.
**Figure** 10 is an XRPD pattern of benzene sulfonate crystal form J.
**Figure** 11 is an XRPD pattern of malonate crystal form K.
**Figure** 12 is an XRPD pattern of hemifumarate crystal form L.
**Figure** 13 is a DSC/TGA profile of free crystal form A.
**Figure** 14 is a DSC/TGA profile of fumarate crystal form B.
**Figure** 15 is a DSC/TGA profile of hydrochloride crystal form C.
**Figure** 16 is a DSC/TGA profile of sulfate crystal form D.
**Figure** 17 is a DSC/TGA profile of succinate crystal form E.
**Figure** 18 is a DSC/TGA profile of malate crystal form F.
**Figure** 19 is a DSC/TGA profile of phosphate crystal form G.
**Figure** 20 is a DSC/TGA profile of **tartrate crystal form H.**
**Figure** 21 is a DSC/TGA profile of pyroglutamate crystal form I.
Figure 22 is a DSC/TGA profile of benzene sulfonate crystal form J.
**Figure** 23 is a DSC/TGA profile of malonate crystal form K.
**Figure** 24 is a DSC/TGA profile of hemifumarate crystal form L.
**Figure** 25 is a DVS profile of free crystal form A.
**Figure** 26 is a DVS profile of fumarate crystal form B.
**Figure** 27 is a DVS profile of hydrochloride crystal form C.
**Figure** 28 is a DVS profile of sulfate crystal form D.
**Figure** 29 is a DVS profile of succinate crystal form E.
**Figure** 30 is a DVS profile of malate crystal form F.
**Figure** 31 is a DVS profile of **different** salt crystal forms.

### Detailed description

Through extensive and in-depth research, the present inventors have unexpectedly discovered, for the first time, an N-substituted phenylsulfonamide compound, or a pharmaceutically acceptable salt thereof, or the solid forms thereof. The salt crystal forms of the present invention significantly enhance the solubility of the compound, thereby facilitating improvement of its physicochemical properties and pharmacokinetic characteristics. The present invention has been accomplished on this basis.

### Terms

Unless otherwise clearly defined herein, all other technical and scientific terms used herein have meanings commonly understood by those skilled in the art in the field to which the present invention belongs.

As used herein, the terms "include", "comprise" and "contain" are used interchangeably to include not only closed definitions, but also semi-closed, and open definitions. In other words, the term includes "consist of" and "substantially consist of".

As used herein, when referring to specifically recited numerical values, the term "about" means that the value can vary from the recited value by no more than 1%. Such as used herein, the expression "about 100" includes all values between 99 and 101 (e.g., 99.1, 99.2, 99.3, 99.4, etc.).

As used herein, the term "room temperature" refers specifically to 25°C ± 5°C.

### Pharmaceutical Compositions and Administration

The solid forms of the present invention, as well as pharmaceutical compositions containing the solid forms as the principal active ingredient, can be used for the treatment, prevention, and alleviation of TRPA1-related disorders.

The pharmaceutical composition of the present invention comprises a safe and effective amount of a compound of the present invention or a pharmacologically acceptable salt thereof, and a pharmacologically acceptable excipient or carrier. In which, "safe and effective amount" is meant that the amount of the compound is sufficient to significantly improve the condition without causing serious side effects. Generally, the pharmaceutical composition contains 0.1-1000 mg of the compound of the present invention/dose, more preferably, 0.5-500 mg of the compound of the present invention/dose. Preferably, the "dose" is a capsule or tablet.

The pharmaceutical composition of the present invention may further incorporate one or more pharmaceutically acceptable carriers. These carriers include conventional pharmaceutical diluents, excipients, fillers, binders, wetting agents, disintegrants, absorption enhancers, surfactants, adsorbent carriers, and lubricants. Pharmaceutically acceptable carriers comprising cellulose and its derivatives thereof (e.g., sodium carboxymethyl cellulose, sodium ethyl cellulose, cellulose acetate, etc.), gelatin, talc, solid lubricants (e.g., stearic acid, magnesium stearate), calcium sulfate, vegetable oils (e.g., soybean oil, sesame oil, peanut oil, olive oil), polyols (e.g., propylene glycol, glycerol, mannitol, sorbitol), emulsifiers (e.g., Tween^{®}), wetting agents (e.g., sodium lauryl sulfate), coloring agents, flavoring agents, stabilizers, antioxidants, preservatives, and pyrogen-free water, etc.

The administration mode of the compound or pharmaceutical composition of the present invention is not particularly limited and representative administration modes include, but are not limited to, oral, rectal, parenteral (intravenous, intramuscular), and topical administration, with oral administration being the preferred route.

The pharmaceutical compositions of the present invention may be formulated as oral, topical or injectable preparations. Representative solid dosage forms for oral administration include capsules, tablets, pills, powders and granules. In these solid dosage forms, the active compounds are mixed with at least one conventional inert excipient (or carrier), such as sodium citrate or dicalcium phosphate, or mixed with any of the following components: (a) fillers or compatibilizer, such as starch, lactose, sucrose, glucose, mannitol and silicic acid; (b) binders, such as hydroxylmethyl cellulose, alginate, gelatin, polyvinylpyrrolidone, sucrose and arabic gum; (c) humectant, such as glycerol; (d) disintegrating agent, such as agar, calcium carbonate, potato starch or tapioca starch, alginic acid, certain composite silicates, and sodium carbonate; (e) dissolution-retarding agents, such as paraffin; (f) absorption accelerators, such as quaternary ammonium compounds; (g) wetting agents, such as cetyl alcohol and glyceryl monostearate; (h) adsorbents, such as kaolin; and (i) lubricants such as talc, stearin calcium, magnesium stearate, solid polyethylene glycol, lauryl sodium sulfate, or the mixtures thereof. In capsules, tablets and pills, the dosage forms may also contain buffering agents.

Solid dosage forms such as tablets, dragees, capsules, pills, and granules can be prepared with coatings and shells such as enteric coatings and other materials known in the art. These dosage forms may optionally contain opacifying agents.

Liquid dosage forms of oral administration include pharmaceutically acceptable emulsions, solutions, suspensions, syrups, or tinctures. In addition to the active compound, the liquid formulations may contain inert diluents conventionally used in the field, such as water or other solvents, solubilizing agents, and emulsifiers, such as thanol, isopropanol, ethyl carbonate, ethyl acetate, propylene glycol, 1,3-butanediol, dimethylformamide and oils, particularly cottonseed oil, peanut oil, corn germ oil, olive oil, castor oil, and sesame oil or mixtures of these substances.

In addition to these inert diluents, the compositions may contain adjuvants such as wetting agents, emulsifying and suspending agents, sweetening agents, flavoring agents and spices.

In addition to the active compound, the suspension may contain suspending agent, such as ethoxylated isooctadecanol, polyoxyethylene sorbitol and dehydrated sorbitan ester, microcrystalline cellulose, aluminum methoxide and agar, or the mixture thereof etc.

The compositions for parenteral injection may comprise physiologically acceptable sterile aqueous or anhydrous solutions, dispersions, suspensions or emulsions and sterile powders which can be re-dissolved into sterile injectable solutions or dispersions. Suitable aqueous and non-aqueous carriers, diluents, solvents or excipients include water, ethanol, polyols and any suitable mixtures thereof.

The solid form of the present invention may be administered alone or in combination with other agents for the prevention and/or treatment of TRPA1-related diseases.

When the composition is application, a safe and effective dose of the cell-free fat extract in the present disclosure is applied to humans or non-human animals (such as rats, mice, dogs, cats, cows, sheep, chickens, and ducks) in need of treatment, where the applied dosage is an acceptable and effective dose in terms of pharmacy, food, or health products. As used herein, the term "safe and effective dose" refers to a dosage that produces a function or activity in humans and/or animals and is acceptable to the humans and/or animals. Those skilled in the art should understand that the "safe and effective dose" may vary with a form of a medication composition, an administration route, excipients of a medication used, severity of a disease, and combined use with other medications. Such as for a human weighing 60 kg, a daily dosage is usually 0.1-1000 mg, preferably 1-600 mg, and more preferably 2-300 mg. Of course, a specific dosage should also consider factors such as the administration route and patient's health status, which are within a skill scope of a skilled physician.

### Advantages of the present invention include

The salt crystal forms of the present invention show excellent solubility properties and stability, thereby facilitating improvement of its physicochemical properties and pharmacokinetic characteristics.

The present invention will be further illustrated below with reference to the specific example. It should be understood that these examples are only to illustrate the invention but not to limit the scope of the invention. Experimental methods in which the specific conditions are not specified in the following examples are usually in accordance with conventional conditions or in accordance with the conditions recommended by the manufacturer.

### Analytical Methods

XRPD: X-ray Powder Diffraction; DSC: Differential Scanning Calorimetry; TGA: Thermogravimetric Analysis; DVS: Dynamic Vapor Sorption.

X-ray powder diffraction analysis: PANalytical X-ray powder diffraction analyzer, working voltage: 45 kV, working current: 40 mA, X-ray powder diffraction patterns were obtained using a Cu target.

Differential scanning calorimetry (DSC) analysis: the instrument is a TA Q2000/Discovery DSC2500; scanning speed: 10° C/min; protective gas, nitrogen.

Thermogravimetric analysis (TGA) analysis: the instrument is a TA Q5000/Discovery TGA5500; scanning speed: 10° C./min; protective gas, nitrogen.

Dynamic moisture adsorption (DVS) analysis: the instrument is a DVS Intrinsic produced by SMS company (Surface Measurement Systems); temperature, 25° C.; carrier gas, flow rate: nitrogen, 200 mL/min; mass change per unit time: 0.002%/min; relative humidity range: 0%RH-95%RH.

Nuclear magnetic resonance analysis: the instrument is a Bruker 400 M nuclear magnetic resonance spectrometer.

The purity of high-performance liquid chromatography (HPLC) was collected on an Agilent 1260 high performance liquid chromatograph.

Ion Chromatography (IC) testing of counter ions to determine molar ratios was collected on a Thermo ICS1100.

### Example 1: free crystal form A of N-(2-(Furan-2-yl)-4-((methylamino)methyl)phenyl)thiophene-3-sulfonamide (I)

According to the method described in Patent Application No. 202110666168.6, *N*-(2-(furan-2-yl)-4-((methylamino)methyl)phenyl)thiophene-3-sulfonamide was prepared. After completing the reaction, the mixture was cooled to 0-10°C, and a solution of purified water and sodium hydroxide aqueous solution was added dropwise. The mixture was filtered, and the filter cake was washed twice with purified water followed by two washes with anhydrous methanol. The resulting filter cake was dried under reduced pressure to obtain a yellow crude compound. The crude product was transferred to a reactor, dissolved in 2 volumes of dimethyl sulfoxide (DMSO), and heated to 60-70°C with stirring for 0.5 hours until the solution clarified. Subsequently, 3 volumes of anhydrous methanol were added dropwise at 60-65°C, followed by stirring for 0.5 hours. The heating was turned off, and the mixture was slowly cooled to 0-10°C, maintained at this temperature for 4 hours with stirring, and then filtered. The obtained yellow free crystal form A was collected with a yield of 79.8%.

### Example 2: Characterization of free crystal form A of N-(2-(Furan-2-yl)-4-((methylamino)methyl)phenyl)thiophene-3-sulfonamide (I)

The compound *N*-(2-(furan-2-yl)-4-((methylamino)methyl)phenyl)thiophene-3-sulfonamide exists in a crystalline state and is designated as free crystal form A. The XRPD pattern is shown in Table 1 and Figure 1. The TGA/DSC overlay patterns are presented in Figure 13. Upon heated to 150°C, the sample exhibited a 0.9% weight loss, with endothermic peaks observed at 181.0°C, 181.8°C (peak temperature), and 182.3°C.

**TABLE 1**

| 2θ | d value | Relative intensity % |
|---|---|---|
| 10.3508 | 8.55 | 3.91 |
| 12.2743 | 7.21 | 5.71 |
| 12.7358 | 6.95 | 2.74 |
| 13.0498 | 6.78 | 3.37 |
| 13.5519 | 6.53 | 5.11 |
| 14.5598 | 6.08 | 3.29 |
| 16.2435 | 5.46 | 12.37 |
| 16.9947 | 5.22 | 2.47 |
| 18.7105 | 4.74 | 5.18 |
| 19.2294 | 4.62 | 19.76 |
| 20.5157 | 4.33 | 3.72 |
| 21.3650 | 4.16 | 4.74 |
| 22.7773 | 3.90 | 8.18 |
| 23.1731 | 3.84 | 14.42 |
| 24.4467 | 3.64 | 100.00 |
| 25.6010 | 3.48 | 4.09 |
| 25.9685 | 3.43 | 3.63 |
| 26.5486 | 3.36 | 3.12 |
| 27.2523 | 3.27 | 1.16 |
| 28.4973 | 3.13 | 0.82 |
| 29.1448 | 3.06 | 0.55 |
| 31.2276 | 2.86 | 1.09 |
| 32.0955 | 2.79 | 1.51 |
| 32.7583 | 2.73 | 12.62 |
| 33.7741 | 2.65 | 1.44 |
| 36.6494 | 2.45 | 1.14 |
| 38.6393 | 2.33 | 0.90 |
| 38.9834 | 2.31 | 0.96. |

### Example 3: Salt and Polymorph Screening of N-(2-(Furan-2-yl)-4-((methylamino)methyl)phenyl)thiophene-3-sulfonamide

The inventors of the present invention conducted salt screening of *N*-(2-(furan-2-yl)-4-((methylamino)methyl)phenyl)thiophene-3-sulfonamide via stirred suspension using a range of acids. The acids selected for screening including hydrochloric acid, sulfuric acid, maleic acid, phosphoric acid, fumaric acid, L-tartaric acid, citric acid, D-glucuronic acid, L-malic acid, succinic acid, pyroglutamic acid, *p*-toluenesulfonic acid, methanesulfonic acid, benzenesulfonic acid, acetic acid, malonic acid, benzoic acid, and hippuric acid. Most acid ligands formed stable salt crystal forms with the target compound. The resulting salt samples were characterized by TGA and DSC. The stoichiometric ratios of the salts were determined using HPLC coupled with IC or ¹H NMR.

### Preparation of Fumarate crystal form B

A mixture of 300.0 mg (0.86 mmol) of the compound (preferably free crystal form A obtained from Example 1) and 101.0 mg fumaric acid (0.87 mmol) was dissolved in 1 mL MeOH. The solution was stirred at room temperature under magnetic agitation, and then transferred to a low-temperature environment and stirred for 1 day. The solid was collected by vacuum filtration, and vacuum-dried to constant weight to obtain 305.2 mg of solid with a yield of 76.1%.

The XRPD data for the fumarate crystal form B are listed in **Table 2**, with the corresponding XRPD pattern shown in **Figure 2**. The TGA/DSC overlay patterns are shown in **Figure 14**.

**TABLE 2**

| 2θ | d value | Relative intensity % |
|---|---|---|
| 4.7853 | 18.47 | 7.44 |
| 11.3360 | 7.81 | 95.62 |
| 12.1412 | 7.29 | 3.80 |
| 12.6401 | 7.00 | 17.06 |
| 13.0507 | 6.78 | 24.77 |
| 13.4538 | 6.58 | 10.32 |
| 14.3983 | 6.15 | 80.83 |
| 15.5359 | 5.70 | 6.50 |
| 16.1411 | 5.49 | 5.45 |
| 16.7535 | 5.29 | 34.94 |
| 18.0815 | 4.91 | 8.61 |
| 19.2348 | 4.61 | 67.67 |
| 19.7074 | 4.50 | 100.00 |
| 19.8575 | 4.47 | 51.16 |
| 22.2983 | 3.99 | 12.31 |
| 22.4667 | 3.96 | 20.37 |
| 22.7769 | 3.90 | 12.17 |
| 23.1803 | 3.84 | 20.68 |
| 24.0261 | 3.70 | 47.03 |
| 24.4094 | 3.65 | 34.43 |
| 24.8487 | 3.58 | 6.41 |
| 25.2842 | 3.52 | 12.97 |
| 25.4446 | 3.50 | 20.09 |
| 25.7526 | 3.46 | 14.15 |
| 25.9064 | 3.44 | 16.01 |
| 26.2756 | 3.39 | 28.27 |
| 26.8582 | 3.32 | 4.14 |
| 27.2796 | 3.27 | 13.01 |
| 28.4514 | 3.14 | 10.46 |
| 28.7628 | 3.10 | 4.24 |
| 29.0176 | 3.08 | 6.10 |
| 31.5419 | 2.84 | 2.31 |
| 32.5956 | 2.75 | 1.81 |
| 34.2989 | 2.61 | 3.71 |
| 35.1125 | 2.56 | 2.28 |
| 36.6111 | 2.45 | 4.94 |
| 38.1686 | 2.36 | 2.94. |

As shown in Figure 14, the DSC pattern reveals an endothermic peak for the fumarate crystal form B near 189.4°C (peak temperature) and has a weight loss of ~0.8% when the fumarate crystal form B is heated to 150°C. Further HPLC/IC analysis confirms the molar ratio of free base to acid as 1:1.

### Preparation of Hydrochloride crystal form C

A total of 500.10 mg (1.43 mmol) of the compound was weighed and dissolved in 6.25 mL tetrahydrofuran (THF). The solution was vortexed and sonicated to ensure complete dissolution. A volume of 167.8 µL (2.00 mmol) of concentrated hydrochloric acid (mass fraction 36-38%) was diluted in 6.25 mL THF, and the hydrochloric THF solution was slowly added dropwise to the stirred suspension of free-state sample under ambient conditions. The mixture was stirred at room temperature for 1 hour and performed suction filtration. The filter cake was dried under vacuum to constant weight to obtain 466.95 mg of solid with a yield of 84.54%.

The XRPD data for the hydrochloride crystal form C are listed in **Table 3**, with the XRPD pattern shown in **Figure 3**. The TGA/DSC overlay patterns are shown in **Figure 15**.

**TABLE 3**

| 2θ | d value | Relative intensity % |
|---|---|---|
| 5.7200 | 15.45 | 17.27 |
| 11.5634 | 7.65 | 41.03 |
| 12.3017 | 7.20 | 17.99 |
| 12.5546 | 7.05 | 10.68 |
| 13.4901 | 6.56 | 24.98 |
| 15.5870 | 5.69 | 11.15 |
| 17.2012 | 5.16 | 57.03 |
| 18.5812 | 4.78 | 6.28 |
| 19.6324 | 4.52 | 21.15 |
| 19.9419 | 4.45 | 19.54 |
| 20.3449 | 4.37 | 46.68 |
| 21.7316 | 4.09 | 3.33 |
| 22.2954 | 3.99 | 3.07 |
| 22.6986 | 3.92 | 16.28 |
| 23.2678 | 3.82 | 28.29 |
| 23.5211 | 3.78 | 38.88 |
| 24.1472 | 3.69 | 9.29 |
| 24.5064 | 3.63 | 12.86 |
| 24.7389 | 3.60 | 41.17 |
| 25.2526 | 3.53 | 100.00 |
| 27.1600 | 3.28 | 13.12 |
| 27.5883 | 3.23 | 8.87 |
| 28.3363 | 3.15 | 10.35 |
| 29.6184 | 3.02 | 18.15 |
| 30.5745 | 2.92 | 2.69 |
| 31.5019 | 2.84 | 5.16 |
| 32.2586 | 2.78 | 4.79 |
| 34.2101 | 2.62 | 2.77 |
| 34.8483 | 2.57 | 2.61 |
| 35.5568 | 2.52 | 5.45 |
| 37.2395 | 2.41 | 2.93 |
| 37.8099 | 2.38 | 2.10 |
| 38.4821 | 2.34 | 1.72 |
| 39.0207 | 2.31 | 0.63. |

As shown in **Figure 15**, the sample has **endothermic peaks** near **93.1°C and 150.7°C (peak temperatures)** and a **weight loss of ~4.2%** when heated to **100°C.** Further **HPLC/IC analysis** confirms the molar ratio of free base to acid as 1:1.

### Preparation of Sulfate crystal form D

A total of 499.63 mg (1.43 mmol) of the compound was weighed and dissolved in 6.25 mL of acetonitrile/water (v/v = 19:1). The solution was vortexed and sonicated to ensure complete dissolution. A volume of 91.8 µL (1.72 mmol) of concentrated sulfuric acid (mass fraction 95-98%) was diluted in 6.25 mL of acetonitrile/water (v/v = 19:1), and the acidic mixture was slowly added dropwise to the stirred suspension of free-state sample under ambient conditions. Once the sample dissolved completely, 25 mL of MTBE was gradually added to the system under stirring to induce solid precipitation. The mixture was stirred at room temperature for approximately 1 hour, performed suction filtration. The filter cake was dried under vacuum to constant weight to obtain 557.98 mg of solid with a yield of 87.08%.

The XRPD data for the pattern for sulfate crystal form D are listed in **Table 4**, with the corresponding XRPD pattern shown in **Figure 4**. The TGA/DSC overlay patterns are shown in **Figure 16****.**

**TABLE 4**

| 2θ | d value | Relative intensity % |
|---|---|---|
| 11.5141 | 7.69 | 11.47 |
| 12.1280 | 7.30 | 9.26 |
| 15.0641 | 5.88 | 2.81 |
| 16.8395 | 5.27 | 22.93 |
| 19.1425 | 4.64 | 6.24 |
| 19.8417 | 4.47 | 3.29 |
| 20.4232 | 4.35 | 5.69 |
| 21.2298 | 4.19 | 4.92 |
| 23.0785 | 3.85 | 13.89 |
| 23.4929 | 3.79 | 10.59 |
| 24.3760 | 3.65 | 100.00 |
| 28.6343 | 3.12 | 2.34 |
| 29.4467 | 3.03 | 3.32. |

As shown in Figure 16, the sample has an endothermic peak near 175.8°C (peak temperature) and a weight loss of about 0.6% when heated to 150°C. Further HPLC/IC analysis confirms the molar ratio of free base to acid as 1:1.

### Preparation of Succinate crystal form E

A total of 300.3 mg (0.86 mmol) of the compound and 102.7 mg (0.87 mmol) of succinic acid were weighed and then sequentially dissolved in 2 mL of methanol. The mixture was stirred at room temperature for 24 hours, and performed suction filtration. The filter cake was dried under vacuum to constant weight to obtain 370.0 mg of solid with a yield of 91.8%.

The XRPD data for the succinate crystal form E are listed in Table 5, with the corresponding XRPD pattern shown in Figure 5. The TGA/DSC overlay patterns are shown in Figure 17.

**TABLE 5**

| 2θ | d value | Relative intensity % |
|---|---|---|
| 11.3012 | 7.83 | 4.51 |
| 12.1022 | 7.31 | 29.55 |
| 12.5682 | 7.04 | 100.00 |
| 12.8251 | 6.90 | 15.88 |
| 14.6341 | 6.05 | 12.00 |
| 14.8890 | 5.95 | 41.52 |
| 15.4251 | 5.74 | 28.69 |
| 16.8216 | 5.27 | 3.36 |
| 17.6695 | 5.02 | 5.00 |
| 18.0351 | 4.92 | 14.38 |
| 18.3497 | 4.84 | 15.48 |
| 18.9745 | 4.68 | 31.08 |
| 19.1778 | 4.63 | 76.32 |
| 19.8916 | 4.46 | 52.13 |
| 21.5010 | 4.13 | 3.16 |
| 21.9794 | 4.04 | 22.25 |
| 22.3705 | 3.97 | 7.90 |
| 22.6780 | 3.92 | 66.97 |
| 22.9693 | 3.87 | 20.66 |
| 23.4757 | 3.79 | 50.03 |
| 24.3137 | 3.66 | 42.93 |
| 25.1547 | 3.54 | 17.11 |
| 25.5114 | 3.49 | 13.68 |
| 25.8422 | 3.45 | 6.55 |
| 26.5689 | 3.36 | 20.74 |
| 27.3275 | 3.26 | 13.09 |
| 27.8768 | 3.20 | 6.11 |
| 28.5630 | 3.13 | 1.87 |
| 29.5834 | 3.02 | 3.10 |
| 30.0966 | 2.97 | 11.09 |
| 31.1706 | 2.87 | 1.74 |
| 31.9845 | 2.80 | 6.84 |
| 33.2344 | 2.70 | 3.96 |
| 34.3892 | 2.61 | 8.84 |
| 34.8567 | 2.57 | 3.40 |
| 36.8940 | 2.44 | 6.24 |
| 38.3053 | 2.35 | 1.89 |
| 38.9442 | 2.31 | 0.78. |

As shown in Figure 17, the sample has an endothermic peak near 172.9°C (peak temperature) and a weight loss of approximately 1.2% when heated to 150°C. Further HPLC/IC analysis confirms the molar ratio of free base to acid as 1:1.

### Preparation of Malate crystal form F:

A total of 300.1 mg (0.86 mmol) of the compound and 117.0 mg (0.87 mmol) of malic acid were weighed and dissolved in 3 mL of tetrahydrofuran. The mixture was stirred at room temperature for 24 hours and performed suction filtration. The filter cake was dried under vacuum to constant weight to obtain 315.4 mg of solid with a yield of 75.6%.

The XRPD data for the malate crystal form F are listed in Table 6, with the corresponding XRPD pattern shown in Figure 6. The TGA/DSC overlay patterns are shown in Figure 18.

**TABLE 6**

| 2θ | d value | Relative intensity % |
|---|---|---|
| 11.4110 | 7.75 | 13.78 |
| 12.1109 | 7.31 | 16.79 |
| 12.6952 | 6.97 | 45.24 |
| 14.5657 | 6.08 | 99.82 |
| 15.4788 | 5.72 | 5.87 |
| 17.7330 | 5.00 | 9.05 |
| 18.3939 | 4.82 | 27.67 |
| 19.1258 | 4.64 | 100.00 |
| 19.4667 | 4.56 | 64.62 |
| 20.8204 | 4.27 | 2.82 |
| 21.4947 | 4.13 | 4.20 |
| 22.1669 | 4.01 | 9.76 |
| 22.4900 | 3.95 | 14.16 |
| 22.9372 | 3.88 | 42.20 |
| 23.7676 | 3.74 | 34.35 |
| 24.3523 | 3.66 | 28.65 |
| 25.5641 | 3.48 | 14.56 |
| 26.4387 | 3.37 | 18.92 |
| 26.8956 | 3.32 | 5.67 |
| 27.4865 | 3.25 | 7.78 |
| 28.0766 | 3.18 | 8.03 |
| 28.6498 | 3.12 | 3.90 |
| 29.3684 | 3.04 | 3.81 |
| 30.3043 | 2.95 | 3.30 |
| 31.3934 | 2.85 | 2.83 |
| 32.2886 | 2.77 | 2.58 |
| 32.9310 | 2.72 | 3.29 |
| 33.2893 | 2.69 | 2.07 |
| 34.7057 | 2.58 | 5.70 |
| 37.2071 | 2.42 | 5.47 |
| 38.7947 | 2.32 | 2.11. |

As shown in Figure 18, the sample has peaks near 144.7°C and 160.6°C (peak temperatures), and shows a weight loss of approximately 2.0% upon heated to 100°C. Further HPLC/IC analysis confirms the molar ratio of free base to acid as 1:1.

### Preparation of Phosphate crystal form G:

A total of 20 mg (0.06 mmol) of the compound and 6.9 mg of phosphoric acid were weighed and dissolved in 0.5 mL of tetrahydrofuran. The mixture was stirred at room temperature and performed suction filtration. The filter cake was dried under vacuum to constant weight to obtain 18.5 mg of solid with a yield of 69.1%.

The XRPD data for the phosphate crystal form G are listed in **Table 7**, with the corresponding XRPD pattern shown in **Figure 7**. The TGA/DSC overlay patterns are shown in **Figure 19****.**

**TABLE 7**

| 2θ | d value | Relative intensity % |
|---|---|---|
| 7.4599 | 11.85 | 5.86 |
| 9.6600 | 9.16 | 4.13 |
| 11.2049 | 7.90 | 100.00 |
| 12.7911 | 6.92 | 22.27 |
| 13.6544 | 6.49 | 3.83 |
| 14.5933 | 6.07 | 7.77 |
| 16.4246 | 5.40 | 6.15 |
| 17.3841 | 5.10 | 1.83 |
| 18.3410 | 4.84 | 2.68 |
| 19.7030 | 4.51 | 52.98 |
| 20.3743 | 4.36 | 10.64 |
| 21.2387 | 4.18 | 40.74 |
| 22.4860 | 3.95 | 49.99 |
| 22.9114 | 3.88 | 7.63 |
| 23.3249 | 3.81 | 23.76 |
| 24.0169 | 3.71 | 3.10 |
| 24.6385 | 3.61 | 10.73 |
| 25.0592 | 3.55 | 7.00 |
| 25.3195 | 3.52 | 6.97 |
| 26.0165 | 3.42 | 4.74 |
| 26.9078 | 3.31 | 3.03 |
| 28.1578 | 3.17 | 9.34 |
| 29.0480 | 3.07 | 1.79 |
| 30.1118 | 2.97 | 17.84 |
| 30.7852 | 2.90 | 3.22 |
| 31.7979 | 2.81 | 7.97 |
| 34.6909 | 2.59 | 2.08 |
| 37.9228 | 2.37 | 2.82. |

As shown in Figure 19, the sample has an endothermic peak near 164.1°C (peak temperature), and indicates a weight loss of approximately 1.8% upon heated to 150°C. Further HPLC/IC analysis confirms the molar ratio of free base to acid as 1:1.

### Preparation of Tartrate crystal form H:

A total of 20 mg (0.06 mmol) of the compound and 9.0 mg of tartaric acid were weighed and dissolved in 5 mL of tetrahydrofuran. The mixture was stirred at room temperature and performed suction filtration. The filter cake was dried under vacuum to constant weight to obtain 24.4 mg of solid with a yield of 81.6%.

The XRPD data for the tartrate crystal form H are listed in Table 8, with the corresponding XRPD pattern shown in Figure 8. The TGA/DSC overlay patterns are shown in Figure 20.

**TABLE 8**

| 2θ | d value | Relative intensity % |
|---|---|---|
| 9.4501 | 9.36 | 3.00 |
| 11.4519 | 7.73 | 12.58 |
| 12.2140 | 7.25 | 10.34 |
| 12.5744 | 7.04 | 16.86 |
| 12.9151 | 6.85 | 18.46 |
| 14.1904 | 6.24 | 69.10 |
| 14.6289 | 6.06 | 18.37 |
| 17.5049 | 5.07 | 5.74 |
| 18.1662 | 4.88 | 35.82 |
| 18.6402 | 4.76 | 39.64 |
| 18.9495 | 4.68 | 100.00 |
| 21.1009 | 4.21 | 5.21 |
| 22.1089 | 4.02 | 20.99 |
| 22.6438 | 3.93 | 2.77 |
| 23.3031 | 3.82 | 17.75 |
| 23.7026 | 3.75 | 39.96 |
| 24.5396 | 3.63 | 30.08 |
| 25.6815 | 3.47 | 12.75 |
| 26.3527 | 3.38 | 10.20 |
| 26.6114 | 3.35 | 9.14 |
| 27.0443 | 3.30 | 6.23 |
| 27.7255 | 3.22 | 1.92 |
| 28.6003 | 3.12 | 7.72 |
| 29.9503 | 2.98 | 3.52 |
| 30.3960 | 2.94 | 3.88 |
| 31.5252 | 2.84 | 2.91 |
| 32.2115 | 2.78 | 2.18 |
| 32.5255 | 2.75 | 4.12 |
| 33.8337 | 2.65 | 1.02 |
| 34.7938 | 2.58 | 3.98 |
| 36.6121 | 2.45 | 3.29 |
| 38.3299 | 2.35 | 3.41. |

As shown in Figure 20, the sample has endothermic peaks near 164.7°C (peak temperatures), and indicates a weight loss of approximately 0.7% upon heated to 150°C. Further HPLC/IC analysis confirms the molar ratio of free base to acid as 1:1.

### Preparation of Pyroglutamate crystal form I:

A total of weigh 20 mg (0.06 mmol) of the compound and 7.7 mg of pyroglutamic acid were weighed and dissolved in 0.5 mL of tetrahydrofuran. The mixture was stirred at room temperature and performed suction filtration. The filter cake was dried under vacuum to constant weight to obtain 25.1 mg of solid with a yield of 87.6%.

The XRPD data for the **pyroglutamate crystal form** I are listed in Table 9, with the corresponding XRPD pattern shown in Figure 9. The TGA/DSC overlay patterns are shown in Figure 21.

**TABLE 9**

| 2θ | d value | Relative intensity % |
|---|---|---|
| 9.2936 | 9.52 | 61.85 |
| 10.7641 | 8.22 | 100.00 |
| 12.8197 | 6.91 | 1.19 |
| 14.3250 | 6.18 | 23.11 |
| 15.6481 | 5.66 | 8.01 |
| 16.9138 | 5.24 | 15.79 |
| 17.5344 | 5.06 | 11.11 |
| 17.8066 | 4.98 | 49.54 |
| 17.9827 | 4.93 | 85.60 |
| 18.8311 | 4.71 | 16.60 |
| 19.3811 | 4.58 | 12.20 |
| 19.9315 | 4.45 | 42.18 |
| 20.9393 | 4.24 | 8.97 |
| 21.2892 | 4.17 | 13.95 |
| 21.6021 | 4.11 | 30.47 |
| 21.8585 | 4.07 | 33.68 |
| 22.9760 | 3.87 | 28.93 |
| 23.7199 | 3.75 | 51.56 |
| 24.4718 | 3.64 | 3.40 |
| 25.2288 | 3.53 | 2.51 |
| 26.2478 | 3.40 | 10.02 |
| 26.7024 | 3.34 | 6.18 |
| 27.7420 | 3.22 | 7.20 |
| 28.2762 | 3.16 | 11.72 |
| 29.5520 | 3.02 | 2.41 |
| 30.4589 | 2.93 | 3.38 |
| 30.8590 | 2.90 | 23.21 |
| 31.5895 | 2.83 | 2.59 |
| 31.9561 | 2.80 | 5.22 |
| 32.9723 | 2.72 | 10.86 |
| 34.6644 | 2.59 | 2.52 |
| 35.5174 | 2.53 | 5.68 |
| 36.6386 | 2.45 | 1.53 |
| 37.4019 | 2.40 | 1.88 |
| 38.6224 | 2.33 | 4.00. |

As shown in Figure 21, the sample has endothermic peaks near 155.4°C (peak temperatures), and has a weight loss of approximately 1.3 % upon heated to 120°C. Further HPLC/IC analysis confirms the molar ratio of free base to acid as 1:1.

### Preparation of Benzene sulfonate crystal form J

A total of 20 mg (0.06 mmol) of the compound and 9.5 mg of **benzenesulfonic** acid were weighed and dissolved in 0.5 mL of tetrahydrofuran. The mixture was stirred at room temperature and performed suction filtration. The filter cake was dried under vacuum to constant weight to obtain 28.3 mg of solid with a yield of 93.1%.

The XRPD data for the **benzene sulfonate crystal form J** are listed in Table 10, with the corresponding XRPD pattern shown in Figure 10. The TGA/DSC overlay patterns are shown in Figure 22.

**TABLE 10**

| 2θ | d value | Relative intensity % |
|---|---|---|
| 7.8263 | 11.30 | 18.85 |
| 9.7005 | 9.12 | 23.70 |
| 11.5344 | 7.67 | 63.01 |
| 13.6926 | 6.47 | 72.52 |
| 15.7215 | 5.64 | 24.00 |
| 16.7789 | 5.28 | 14.03 |
| 17.9617 | 4.94 | 37.91 |
| 19.4764 | 4.56 | 82.26 |
| 19.9603 | 4.45 | 33.29 |
| 21.0708 | 4.22 | 100.00 |
| 22.1505 | 4.01 | 85.15 |
| 23.0614 | 3.86 | 48.53 |
| 24.6362 | 3.61 | 15.71 |
| 27.6427 | 3.23 | 21.08 |
| 29.5771 | 3.02 | 22.37. |

As shown in Figure 22, the sample has an endothermic peak near 164.9°C (peak temperatures), and has a weight loss of approximately 1.3 % upon heated to 150°C. Further HPLC/IC analysis confirms the molar ratio of free base to acid as 1:1.

### Preparation of Malonate crystal form K

A total of 20 mg (0.06 mmol) of the compound and 6.2 mg of **malonic** acid were weighed and dissolved in 0.5 mL of tetrahydrofuran. The mixture was stirred at room temperature and performed suction filtration. The filter cake was dried under vacuum to constant weight to obtain 21.7 mg of solid with a yield of 80.1%.

The XRPD data for the **malonate crystal form K** are listed in Table 11, with the corresponding XRPD pattern shown in Figure 11. The TGA/DSC overlay patterns are shown in Figure 23.

**TABLE 11**

| 2θ | d value | Relative intensity % |
|---|---|---|
| 11.7720 | 7.52 | 11.86 |
| 12.6542 | 7.00 | 12.02 |
| 13.7132 | 6.46 | 13.76 |
| 15.2846 | 5.80 | 100.00 |
| 15.8543 | 5.59 | 7.35 |
| 16.3506 | 5.42 | 3.99 |
| 18.9714 | 4.68 | 10.90 |
| 19.4289 | 4.57 | 21.60 |
| 19.6560 | 4.52 | 24.43 |
| 20.4179 | 4.35 | 38.71 |
| 22.6553 | 3.92 | 13.46 |
| 23.6581 | 3.76 | 20.02 |
| 24.3848 | 3.65 | 12.06 |
| 25.4604 | 3.50 | 17.25 |
| 26.4165 | 3.37 | 18.93 |
| 27.2939 | 3.27 | 4.65 |
| 27.8164 | 3.21 | 13.05 |
| 30.8357 | 2.90 | 2.01. |

As shown in Figure 23, the sample has an endothermic peak near 139.8°C (peak temperatures), and has a weight loss of approximately 1.6 % upon heated to 130°C. Further HPLC/IC analysis confirms the molar ratio of free base to acid as 1:1.

### Preparation of Hemifumarate crystal form L

A total of 500.8 mg (1.44 mmol) of the compound and 83.6 mg (0.72mmol) of **fumaric acid** were weighed and dissolved in 2.0 mL of methanol. The mixture was stirred at room temperature for 4 days and performed suction filtration. The filter cake was dried under vacuum to constant weight to obtain 489.2 mg of solid with a yield of 83.7%.

The XRPD data for the **hemifumarate crystal form L** are listed in Table 12, with the corresponding XRPD pattern shown in Figure 12. The TGA/DSC overlay patterns are shown in Figure 24.

**TABLE 12**

| 2θ | d value | Relative intensity % |
|---|---|---|
| 5.7467 | 15.38 | 21.65 |
| 8.9553 | 9.87 | 4.23 |
| 11.5652 | 7.65 | 67.24 |
| 12.1205 | 7.30 | 31.74 |
| 12.7752 | 6.93 | 4.53 |
| 13.3790 | 6.62 | 5.53 |
| 13.6634 | 6.48 | 11.42 |
| 14.7284 | 6.01 | 5.86 |
| 15.5191 | 5.71 | 10.25 |
| 16.9116 | 5.24 | 27.22 |
| 17.2506 | 5.14 | 100.00 |
| 17.5018 | 5.07 | 21.59 |
| 18.8042 | 4.72 | 5.64 |
| 19.3060 | 4.60 | 25.92 |
| 20.3231 | 4.37 | 10.70 |
| 20.6655 | 4.30 | 18.63 |
| 21.4639 | 4.14 | 4.44 |
| 22.2232 | 4.00 | 6.22 |
| 22.8905 | 3.89 | 14.42 |
| 23.0787 | 3.85 | 47.91 |
| 23.5842 | 3.77 | 11.85 |
| 23.8077 | 3.74 | 14.53 |
| 24.3257 | 3.66 | 48.70 |
| 24.6192 | 3.62 | 16.58 |
| 25.6766 | 3.47 | 37.11 |
| 27.5081 | 3.24 | 8.66 |
| 28.7002 | 3.11 | 7.19 |
| 29.1791 | 3.06 | 3.76 |
| 30.4714 | 2.93 | 9.77 |
| 32.4509 | 2.76 | 3.66 |
| 33.2324 | 2.70 | 2.78 |
| 35.3682 | 2.54 | 2.19 |
| 35.9013 | 2.50 | 4.06 |
| 36.7589 | 2.45 | 2.58. |

As shown in Figure 24, the sample has endothermic a peak near 173.8°C (peak temperatures), and has a weight loss of approximately 0.3 % upon heated to 150°C. Further HPLC/IC analysis confirms the molar ratio of free base to acid as 1:0.5.

### Example 4: To Assess the Characteristics of the Example Crystal Forms Hygroscopicity

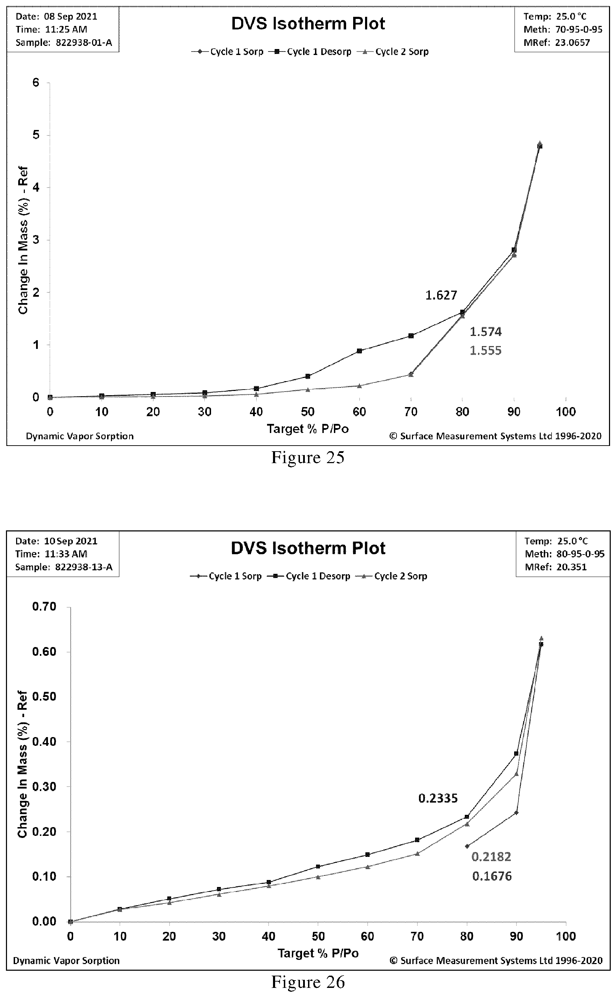

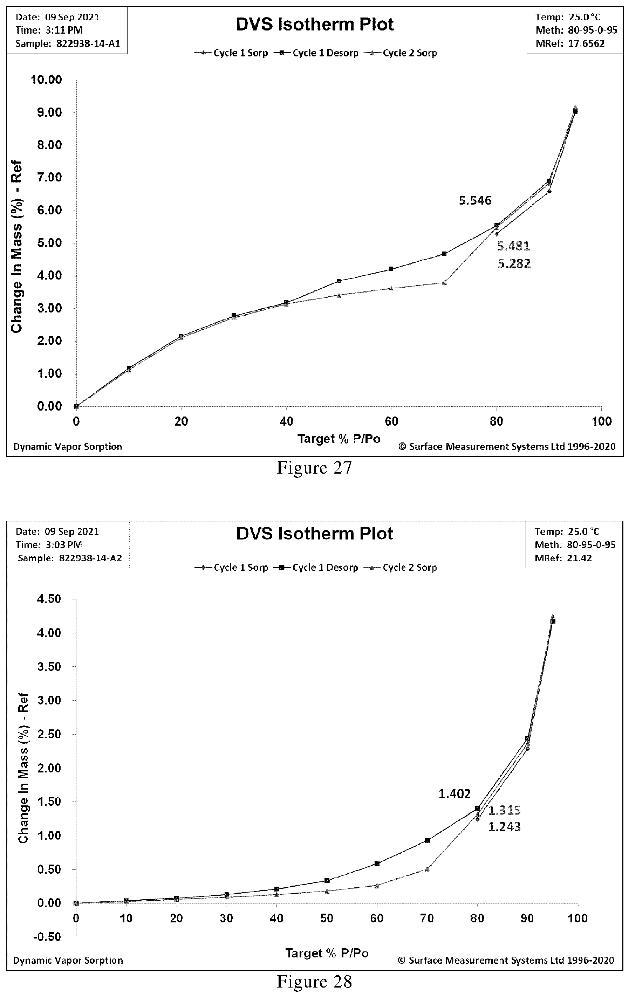

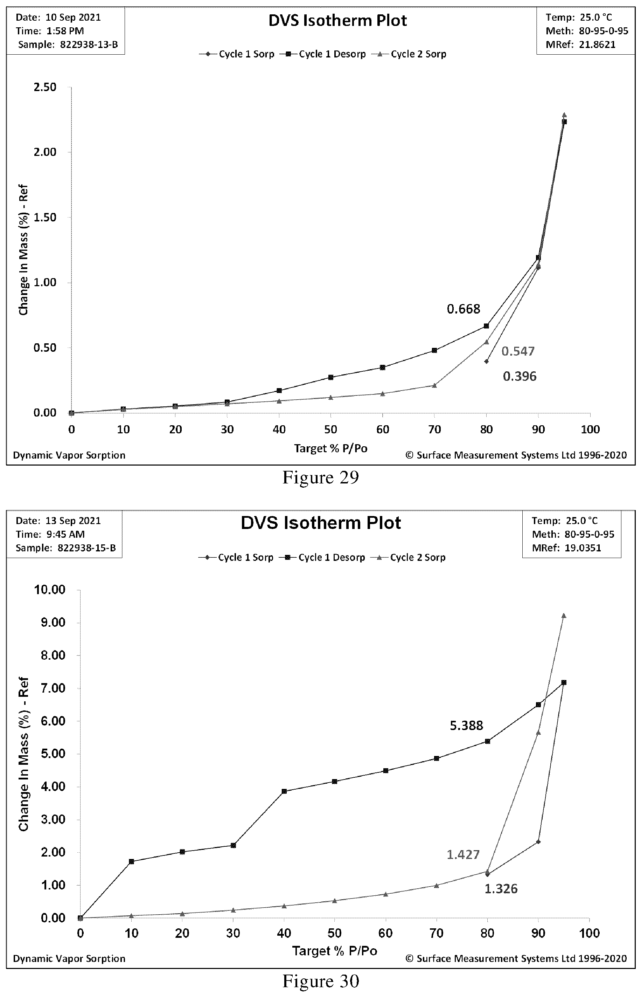

Dynamic moisture absorption (DVS) was used to test their hygroscopicity. The results are shown in Figure 25 to Figure 30. On the 0% RH-95% RH adsorption curves at 25°C/80% RH, the moisture adsorption values were shown as follows: the free crystal form A showed 1.6% moisture uptake (slightly hygroscopic), the fumarate crystal form B exhibited 0.2% (slightly hygroscopic), the hydrochloride crystal form C demonstrated 5.5% (hygroscopic), the sulfate crystal form D displayed 1.3% (slightly hygroscopic), the succinate crystal form E showed 0.6% (slightly hygroscopic), and the malate crystal form F presented 1.4% moisture adsorption (slightly hygroscopic), according to the hygroscopicity classification criteria specified in 2015 edition of the Chinese Pharmacopoeia (Guidelines for Drug Hygroscopicity Test).

### Dynamic Solubility

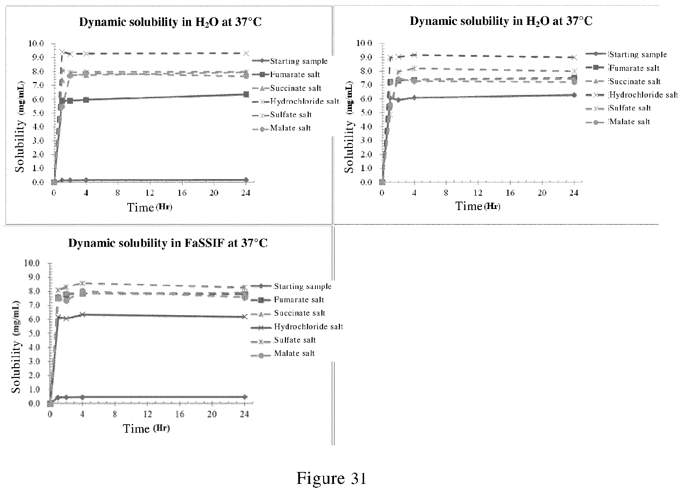

The dynamic solubility of fumarate crystal form B, hydrochloride crystal form C, sulfate crystal form D, succinate crystal form E, malate crystal form F, and free crystal form A was assessed at 37°C in biorelevant media SGF, FaSSIF or water. All samples were dosed at 10 mg/mL in each solvent. The samples were sealed and mounted on a rotating disk rotating at 25 rpm, and the rotating disk was placed in a 37° C. incubator. Samples were taken at time points 1, 2, 4, and 24 hours of equilibration, and the filtrate was separated and tested concentration by HPLC and pH, while isolated solids were characterized by XRPD. As shown in Figure 31, all salt crystal forms demonstrated significantly enhanced solubility compared to the free form. The free form exhibited negligible dissolution in water and FaSSIF, whereas salt crystal forms achieved solubility levels of 6-10 mg/mL.

### Solid-State Stability

About 10 mg of each solid form of the compound was weighed and added to the HPLC vial, respectively, the bottleneck was sealed with a sealing film, and 10 small holes were made on the film. The vial was placed in 25° C/60% RH and 40° C/75% RH environment for 4 weeks, samples were taken in the 1st week, the 2rd week and the 4th week respectively, the purity (by HPLC analysis) and crystal form (by X ray powder diffraction analysis) of the sample were investigated. The HPLC purity did not decrease significantly in the free form and salt crystal forms under the tested storage conditions, and XRPD analysis revealed no detectable crystalline phase changes in the free form and salt crystal forms before and after placement. The solid-state stability results are summarized in Table 13.

**TABLE 13**

| Starting samples | Initial purity (area %) | Environmental Conditions | Environmental Time | Purity (area %) | Crystalline changes |
|---|---|---|---|---|---|
| Free crystal form A | 98.11 | 25°C /60%RH/open | 1 week | 98.18 | NO |
| | | | 2 weeks | 98.14 | NO |
| | | | 4 weeks | 98.1 | NO |
| | | 40°C /75%RH/open | 1 week | 98.19 | NO |
| | | | 2 weeks | 98.2 | NO |
| | | | 4 weeks | 98.13 | NO |
| Fumarate crystal form B | 98.55 | 25°C /60%RH/open | 1 week | 98.53 | NO |
| | | | 2 weeks | 98.47 | NO |
| | | | 4 weeks | 98.57 | NO |
| | | 40°C /75%RH/open | 1 week | 98.4 | NO |
| | | | 2 weeks | 98.5 | NO |
| | | | 4 weeks | 98.52 | NO |
| Hydrochloride crystal form C | 99.02 | 25°C /60%RH/open | 1 week | 98.97 | NO |
| | | | 2 weeks | 98.92 | NO |
| | | | 4 weeks | 98.87 | NO |
| | | 40°C /75%RH/open | 1 week | 99.02 | NO |
| | | | 2 weeks | 98.9 | NO |
| | | | 4 weeks | 99 | NO |
| Sulfate crystal form D | 98.77 | 25°C /60%RH/open | 1 week | 98.74 | NO |
| | | | 2 weeks | 98.65 | NO |
| | | | 4 weeks | 98.78 | NO |
| | | 40°C /75%RH/open | 1 week | 98.76 | NO |
| | | | 2 weeks | 98.65 | NO |
| | | | 4 weeks | 98.78 | NO |
| Succinate crystal form E | 98.82 | 25°C /60%RH/open | 1 week | 98.92 | NO |
| | | | 2 weeks | 98.86 | NO |
| | | | 4 weeks | 98.94 | NO |
| | | 40°C /75%RH/open | 1 week | 98.92 | NO |
| | | | 2 weeks | 98.72 | NO |
| | | | 4 weeks | 98.98 | NO |
| Malate crystal form F | 98.72 | 25°C /60%RH/open | 1 week | 98.72 | NO |
| | | | 2 weeks | 98.7 | NO |
| | | | 4 weeks | 98.79 | NO |
| | | 40°C /75%RH/open | 1 week | 98.74 | NO |
| | | | 2 weeks | 98.75 | NO |
| | | | 4 weeks | 98.68 | NO |
| Phosphate crystal form G | 98.11 | 25°C /60%RH/open | 1 week | 98.11 | NO |
| | | | 2 weeks | 98.09 | NO |
| | | | 4 weeks | 98.04 | NO |
| | | 40°C /75%RH/open | 1 week | 98.11 | NO |
| | | | 2 weeks | 98.06 | NO |
| | | | 4 weeks | 98.01 | NO |
| Tartrate crystal form H | 98.71 | 25°C /60%RH/open | 1 week | 98.71 | NO |
| | | | 2 weeks | 98.62 | NO |
| | | | 4 weeks | 98.6 | NO |
| | | 40°C /75%RH/open | 1 week | 98.7 | NO |
| | | | 2 weeks | 98.6 | NO |
| | | | 4 weeks | 98.53 | NO |
| Pyroglutamate crystal form I | 98.61 | 25°C /60%RH/open | 1 week | 98.61 | NO |
| | | | 2 weeks | 98.58 | NO |
| | | | 4 weeks | 98.57 | NO |
| | | 40°C /75%RH/open | 1 week | 98.61 | NO |
| | | | 2 weeks | 98.54 | NO |
| | | | 4 weeks | 98.32 | NO |
| Benzene sulfonate crystal form J | 98.87 | 25°C /60%RH/open | 1 week | 98.87 | NO |
| | | | 2 weeks | 98.85 | NO |
| | | | 4 weeks | 98.82 | NO |
| | | 40°C /75%RH/open | 1 week | 98.87 | NO |
| | | | 2 weeks | 98.82 | NO |
| | | | 4 weeks | 98.79 | NO |
| Malonate crystal form K | 98.64 | 25°C /60%RH/open | 1 week | 98.64 | NO |
| | | | 2 weeks | 98.61 | NO |
| | | | 4 weeks | 98.59 | NO |
| | | 40°C /75%RH/open | 1 week | 98.64 | NO |
| | | | 2 weeks | 98.58 | NO |
| | | | 4 weeks | 98.46 | NO |

### Solubility

To determine the approximate solubility, the corresponding solid form sample was added to a 3 mL glass vial. Purified water was incrementally introduced (50, 50, 200, and 700 µL) while vortexing and ultrasonically agitating to facilitate dissolution. The mixture was observed to check if the solid completely dissolved. Solvent addition was halted either when full dissolution was achieved or when the total water volume reached 1 mL. The approximate solubility was then calculated based on the final solvent volume used.

The approximate solubility test results are summarized in Table 14. Compared to the free crystal form A, each salt crystal form exhibited a significant increase in solubility in purified water.

**TABLE 14**

| Sample | Solubility (mg/mL) |
|---|---|
| Free form crystal A | S<2.0 |
| Succinate crystal form E | 10.0<S<20.0 |
| Phosphate crystal form G | 10.0<S<20.0 |
| **Tartrate crystal form H** | 10.0<S<20.0 |
| Pyroglutamate crystal form I | S>38.0 |
| Malonate crystal form K | 10.0<S<20.0 |

All documents mentioned herein are incorporated by reference in this application as if each document were individually incorporated by reference. In addition, it should be understood that after reading the above teaching content of the present invention, those skilled in the art can make various changes or modifications to the present invention, and these equivalent forms also fall within the scope defined by the appended claims of the present application.

## Claims

1. A solid form of
*N*-(2-(furan-2-yl)-4-((methylamino)methyl)phenyl)thiophene-3-sulfonamide,
the solid form includes free crystal form or salt crystal form;
the salt crystal form is a salt crystal form formed by reacting the compound with a pharmaceutically acceptable acid, and the pharmaceutically acceptable acid is selected from the group consisting of hydrochloric acid, sulfuric acid, maleic acid, phosphoric acid, fumaric acid, L-tartaric acid, citric acid, D-glucuronic acid, L-malic acid, succinic acid, pyroglutamic acid, p-toluenesulfonic acid, methanesulfonic acid, benzenesulfonic acid, acetic acid, malonic acid, benzoic acid and hippuric acid.

2. The solid form of *N*-(2-(furan-2-yl)-4-((methylamino)methyl)phenyl)thiophene-3-sulfonamide of claim 1,
wherein the solid form is free crystal form A, and the X-ray powder diffraction pattern of the free crystal form A has characteristic peaks at the following 2θ values: 16.24±0.2°, 19.23±0.2°, 23.17±0.2°, 24.45±0.2°, and 32.76±0.2°.

3. The solid form of
*N*-(2-(furan-2-yl)-4-((methylamino)methyl)phenyl)thiophene-3-sulfonamide of claim 1, wherein the solid form is fumarate crystal form B, and the X-ray powder diffraction pattern of the fumarate crystal form B has characteristic peaks at the following 2θ values: 11.34±0.2°, 14.40±0.2°, 19.71±0.2°, and 19.86±0.2°.

4. The solid form of
*N*-(2-(furan-2-yl)-4-((methylamino)methyl)phenyl)thiophene-3-sulfonamide of claim 1, wherein the solid form is hydrochloride crystal form C, and the X-ray powder diffraction pattern of the hydrochloride crystal form C has characteristic peaks at the following 2θ values: 17.20±0.2°, 20.34±0.2°, 24.74±0.2°, and 25.25±0.2°.

5. The solid form of
*N*-(2-(furan-2-yl)-4-((methylamino)methyl)phenyl)thiophene-3-sulfonamide of claim 1, wherein the solid form is sulfate crystal form D, and the X-ray powder diffraction pattern of the sulfate crystal form D has characteristic peaks at the following 2θ values: 16.84±0.2°, 23.08±0.2°, and 24.38±0.2°.

6. The solid form of
*N*-(2-(furan-2-yl)-4-((methylamino)methyl)phenyl)thiophene-3-sulfonamide of claim 1, wherein the solid form is succinate crystal form E, and the X-ray powder diffraction pattern of the succinate crystal form E has characteristic peaks at the following 2θ values: 12.57±0.2°, 19.18±0.2°, 19.89±0.2°, and 22.68±0.2°.

7. The solid form of
*N*-(2-(furan-2-yl)-4-((methylamino)methyl)phenyl)thiophene-3-sulfonamide of claim 1, wherein the solid form is malate crystal form F, and the X-ray powder diffraction pattern of the malate crystal form F has characteristic peaks at the following 2θ values: 12.70±0.2°, 14.57±0.2°, 19.13±0.2°, and 19.47±0.2°.

8. The solid form of
*N*-(2-(furan-2-yl)-4-((methylamino)methyl)phenyl)thiophene-3-sulfonamide of claim 1, wherein the solid form is phosphate crystal form G, and the X-ray powder diffraction pattern of the phosphate crystal form G has characteristic peaks at the following 2θ values: 11.20±0.2°, 19.70±0.2°, 21.24±0.2°, and 22.49±0.2°.

9. The solid form of
*N*-(2-(furan-2-yl)-4-((methylamino)methyl)phenyl)thiophene-3-sulfonamide of claim 1, wherein the solid form is tartrate crystal form H, and the X-ray powder diffraction pattern of the tartrate crystal form H has characteristic peaks at the following 2θ values: 14.19±0.2°, 18.64±0.2°, 18.95±0.2°, and 23.70±0.2°.

10. The solid form of
*N*-(2-(furan-2-yl)-4-((methylamino)methyl)phenyl)thiophene-3-sulfonamide of claim 1, wherein the solid form is pyroglutamate crystal form I, the X-ray powder diffraction pattern of the pyroglutamate crystal form I has characteristic peaks at the following 2θ values: 9.29±0.2°, 10.76±0.2°, 17.98±0.2°, and 23.72±0.2°.

11. The solid form of
*N*-(2-(furan-2-yl)-4-((methylamino)methyl)phenyl)thiophene-3-sulfonamide of claim 1, wherein the solid form is benzenesulfonate crystal form J, and the X-ray powder diffraction pattern of the benzenesulfonate crystal form J has characteristic peaks at the following 2θ values: 13.69±0.2°, 19.48±0.2°, 21.07±0.2°, and 22.15±0.2°.

12. The solid form of
*N*-(2-(furan-2-yl)-4-((methylamino)methyl)phenyl)thiophene-3-sulfonamide of claim 1, wherein the solid form is malonate crystal form K, and the X-ray powder diffraction pattern of the malonate crystal form K has characteristic peaks at the following 2θ values: 15.28±0.2°, 19.66±0.2°, and 20.42±0.2°.

13. The solid form of
*N*-(2-(furan-2-yl)-4-((methylamino)methyl)phenyl)thiophene-3-sulfonamide of claim 1, wherein the solid form is hemifumarate crystal form L, and the X-ray powder diffraction pattern of the hemifumarate crystal form L has characteristic peaks at the following 2θ values: 11.57±0.2°, 17.25±0.2°, 23.08±0.2°, and 24.33±0.2°.

14. The solid form of
*N*-(2-(furan-2-yl)-4-((methylamino)methyl)phenyl)thiophene-3-sulfonamide of claim 1, wherein the solid form is fumarate crystal form B, and the X-ray powder diffraction pattern of the fumarate crystal form B has characteristic peaks at the following 2θ values: 11.34±0.2°, 14.40±0.2°, 19.23±0.2°, and 19.71±0.2°.

15. A method for preparing the solid form of
*N*-(2-(furan-2-yl)-4-((methylamino)methyl)phenyl)thiophene-3-sulfonamide of claim 1, wherein the method includes any one of Method One to Eight:
Method One comprises:
(a1) dissolving the prepared crude compound in a first solvent, heating, and mixing until clear;
(a2) adding a second solvent, cooling and crystallizing to obtain the free crystal form A;
Method Two comprises:
dissolving and mixing compound raw material and fumaric acid in a third solvent, cooling and crystallizing to obtain the fumarate crystal form B;
Method Three comprises:
(b1) dissolving compound raw material in a fourth solvent;
(b2) dissolving concentrated hydrochloric acid in a fourth solvent;
(b3) adding the solution of hydrochloric acid in the fourth solvent obtained in step (b2) dropwise into the mixture obtained in step (b1), mixing and crystallizing to obtain the hydrochloride crystal form C;
Method Four comprises:
(c1) dissolving compound raw material in a fifth solvent;
(c2) dissolving concentrated sulfuric acid in a fifth solvent;
(c3) adding the solution of sulfuric acid in the fifth solvent obtained in step (c2) dropwise into the mixture obtained in step (c1), and dissolving to clear;
(c4) adding a sixth solvent dropwise into the mixture obtained in step (c3), mixing and crystallizing to obtain the sulfate crystal form D;
Method Five comprises:
dissolving compound raw material and succinic acid in a seventh solvent, mixing and crystallizing to obtain the succinate crystal form E;
Method Six comprises:
dissolving compound raw material and malic acid in a eighth solvent, mixing and crystallizing to obtain the malate crystal form F;
Method Seven comprises:
dissolving compound raw material and an acid in a ninth solvent, mixing and crystallizing to obtain the phosphate crystal form G, the tartrate crystal form H, the pyroglutamate crystal form I, the benzenesulfonate crystal form J, and the malonate crystal form K that correspond to the acid; wherein the acid is selected from the group consisting of phosphoric acid, tartaric acid, pyroglutamic acid, benzenesulfonic acid, or malonic acid;
Method Eight comprises: dissolving compound raw material and fumaric acid in a third solvent, mixing and crystallizing to obtain the hemifumarate crystal form L;
wherein the compound raw material comprises amorphous or crystalline form of *N*-(2-(furan-2-yl)-4-((methylamino)methyl)phenyl)thiophene-3-sulfonamide.

16. A pharmaceutical composition, comprises the solid form of
N-(2-(furan-2-yl)-4-((methylamino)methyl)phenyl)thiophene-3-sulfonamide of any one of claims 1 to 14; and pharmaceutically acceptable excipients.

17. Use of the solid form of
N-(2-(furan-2-yl)-4-((methylamino)methyl)phenyl)thiophene-3-sulfonamide of any one of claims 1 to 14, wherein for (a) preparing an inhibitor of transient receptor potential channel protein TRPA1; and/or (b) preparing a drug for preventing and/or treating diseases associated with transient receptor potential channel protein TRPA1.

18. The use of claim 17, wherein the disease associated with transient receptor potential channel protein TRPA1 is selected from the group consisting of: inflammatory bowel disease, irritable bowel syndrome, pain, inflammation, and a combination thereof.
